(19) Europäisches Patentamt
European Patent Office
Office européen des brevets

(11) Veröffentlichungsnummer: **0 312 908 A1**

# (12) EUROPÄISCHE PATENTANMELDUNG

(21) Anmeldenummer: **88116980.9**

(22) Anmeldetag: **13.10.88**

(51) Int. Cl.4: **C07C 121/84 , C07C 121/45 , C07C 147/02 , A01N 37/34 , A01N 41/10**

(30) Priorität: **23.10.87 DE 3735902**

(43) Veröffentlichungstag der Anmeldung: **26.04.89 Patentblatt 89/17**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Heidenreich, Holger, Dr.**
**Blankenese 18**
**D-2224 Kuden(DE)**
Erfinder: **Becker, Benedikt, Dr.**
**Metzkausener Strasse 14**
**D-4020 Mettmann(DE)**

## (54) Derivate des 2,3-Diaminomaleinsäurenitrils.

(57) Es wurden neue Derivat des 2,3-Diaminomaleinsäurenitrils der allgemeinen Formel

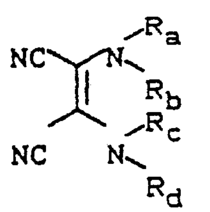

(I)

bereitgestellt,
in welcher
die Substituenten $R_a$, $R_b$, $R_c$ und $R_d$ jeweils eine der folgenden Bedeutungskombinationen A, B, C, D, E oder F annehmen können:

A) $R_a$ und $R_b$ stehen zusammen für den Rest

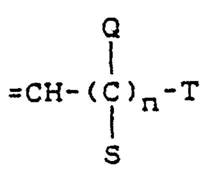

und $R_c$ und $R_d$ stehen zusammen für den Rest

EP 0 312 908 A1

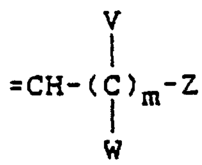

wobei
Q, S, T, V, W und Z gleich oder verschieden sein können und unabhängig voneinander für Wasserstoff, Halogen, gegebenenfalls substituiertes Alkyl, gegebenenfalls substituiertes Aryl, gegebenenfalls substituiertes Alkylsulfonyl oder gegebenenfalls substituiertes Arylsulfonyl stehen,
n und m unabhängig voneinander die Zahlen 0 bis 10 annehmen können,

B) $R_a$ und $R_b$ stehen zusammen für den Rest

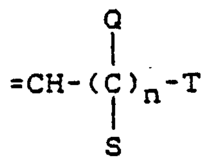

und $R_c$ und $R_d$ stehen zusammen für den Rest

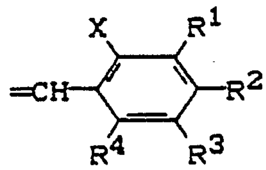

wobei
Q, S, T und n die unter A) angegebene Bedeutung besitzen und
X für Wasserstoff, Halogen, Halogenalkyl oder CN steht,
$R^1$, $R^2$, $R^3$ und $R^4$ gleich oder verschieden sein können und
für Wasserstoff, gegebenenfalls substituiertes Alkyl, gegebenenfalls substituiertes Alkoxy, Halogen, CN, $NO_2$, gegebenenfalls substituiertes Dialkylamino, Alkoxycarbonyl, gegebenenfalls substituiertes Alkylthio, gegebenenfalls substituiertes Alkylthionyl, gegebenenfalls substituiertes Alkylsulfonyl, OH, SH, $NH_2$ stehen,

C) $R_a$ steht für Wasserstoff und $R_b$ steht für den Rest

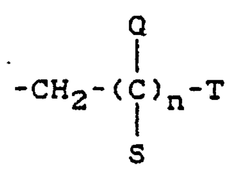

und $R_c$ und $R_d$ stehen zusammen für den Rest

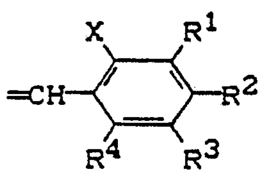

wobei
Q, S, T, n, X, $R^1$, $R^2$, $R^3$ und $R^4$ die unter B) angegebene Bedeutung besitzen,

D) $R_a$ und $R_b$ stehen zusammen für den Rest

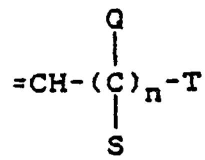

$R_c$ steht für Wasserstoff und $R_d$ steht für den Rest

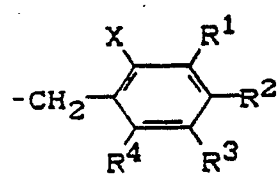

worin
Q, S, T, n, X, $R^1$, $R^2$, $R^3$ und $R^4$ die unter B) angegebene Bedeutung besitzen,

E) $R_a$ und $R_c$ stehen für Wasserstoff, $R_b$ steht für den Rest

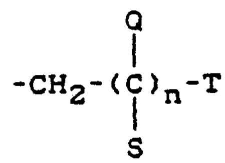

und $R_d$ steht für den Rest

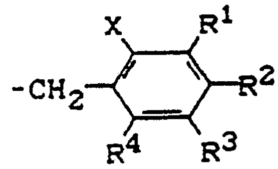

worin
Q, S, T, n, X, $R^1$, $R^2$, $R^3$ und $R^4$ die unter B) angegebene Bedeutung besitzen,

F) $R_a$ und $R_c$ stehen für Wasserstoff, $R_b$ steht für den Rest

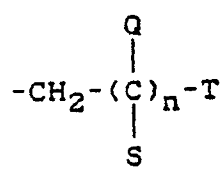

und $R_d$ für den Rest

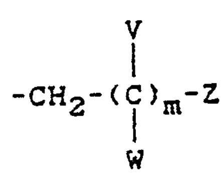

wobei
Q, S, T, V, W, Z, n und m die unter A) angegebene Bedeutung besitzen.

Die erfindungsgemäßen Verbindungen besitzen eine stark ausgeprägte Wirksamkeit gegen tierische Schädlinge und insbesondere gegen Spinnentiere.

## Derivate des 2,3-Diaminomaleinsäurenitrils

Die vorliegende Erfindung betrifft neue Derivate des 2,3-Diaminomaleinsäurenitrils, Verfahren zu ihrer Herstellung und ihre Verwendung als Schädlingsbekämpfungsmittel, insbesondere als Akarizide.

Derivate des 2,3-Diaminomaleinsäurenitrils sind aus verschiedenen Literaturstellen bekannt. Siehe z.B. US-Patentschrift Nr. 4 002 616, wo Bisanil-Derivate beschrieben werden oder siehe z.B. Onoda in Nippon Nogeikagaku Kaishi 36(2), 167-72 (1962) und JP 501 57 320, wo ebenso wie in den Publikationen Robertson, Vanghan in Journal of the American Chemical Society 80, 2691 (1958), Hinkel et al., Journal of the Chemical Society 1937, 1432 und Begland et al., Journal of Organic Chemistry, 39, 2341 (1974), Monoanilderivate von 2,3-Diaminomaleinsäurenitril beschrieben werden.

Über eine Wirksamkeit der vorgenannten Verbindungsklassen oder anderer Derivate des 2,3-Diaminomaleinsäurenitrils gegen tierische Schädlinge ist jedoch bisher noch nichts bekannt geworden.

Es wurde gefunden, daß die neuen Derivate von 2,3-Diaminomaleinsäurenitril der allgemeinen Formel

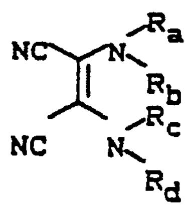

(I)

in welcher
die Substituenten $R_a$, $R_b$, $R_c$ und $R_d$ jeweils eine der folgenden Bedeutungskombinationen A, B, C, D, E oder F
annehmen können:

(A) $R_a$ und $R_b$ stehen zusammen für den Rest

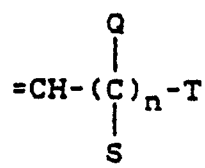

und $R_c$ und $R_d$ stehen zusammen für den Rest

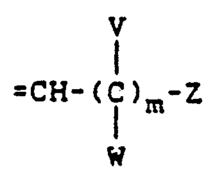

wobei
Q, S, T, V, W und Z gleich oder verschieden sein können und unabhängig voneinander für Wasserstoff, Halogen, gegebenenfalls substituiertes Alkyl, gegebenenfalls substituiertes Aryl, gegebenenfalls substituiertes Alkylsulfonyl oder gegebenenfalls substituiertes Arylsulfonyl stehen,
n und m unabhängig voneinander die Zahlen 0 bis 10 annehmen können,

B) $R_a$ und $R_b$ stehen zusammen für den Rest

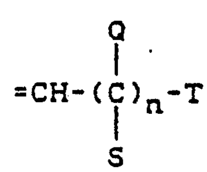

und $R_c$ und $R_d$ stehen zusammen für den Rest

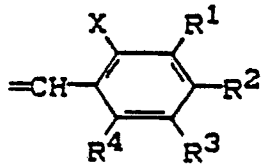

wobei
Q, S, T und n die unter A) angegebene Bedeutung besitzen und
X für Wasserstoff, Halogen, Halogenalkyl oder CN steht,
$R^1$, $R^2$, $R^3$ und $R^4$ gleich oder verschieden sein können und
für Wasserstoff, gegebenenfalls substituiertes Alkyl, gegebenenfalls substituiertes Alkoxy, Halogen, CN, $NO_2$, gegebenenfalls substituiertes Dialkylamino, Alkoxycarbonyl, gegebenenfalls substituiertes Alkylthio, gegebenenfalls substituiertes Alkylthionyl, gegebenenfalls substituiertes Alkylsulfonyl, OH, SH, $NH_2$ stehen,

C) $R_a$ steht für Wasserstoff und $R_b$ steht für den Rest

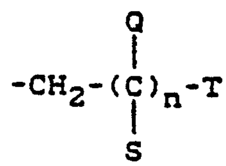

und $R_c$ und $R_d$ stehen zusammen für den Rest

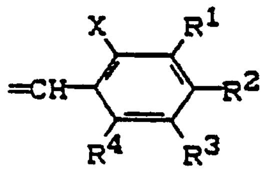

wobei
Q, S, T, n, X, $R^1$, $R^2$, $R^3$ und $R^4$ die unter B) angegebene Bedeutung besitzen,

D) $R_a$ und $R_b$ stehen zusammen für den Rest

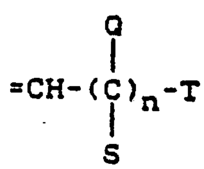

$R_c$ steht für Wasserstoff und $R_d$ steht für den Rest

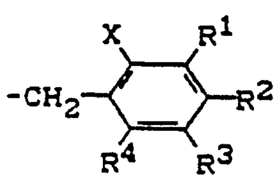

worin
Q, S, T, n, X, $R^1$, $R^2$, $R^3$ und $R^4$ die unter B) angegebene Bedeutung besitzen,

E) $R_a$ und $R_c$ stehen für Wasserstoff, $R_b$ steht für den Rest

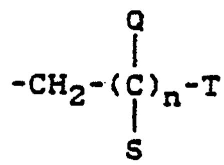

und $R_d$ steht für den Rest

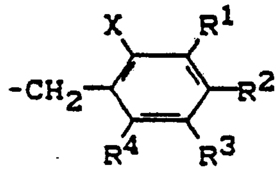

worin
Q, S, T, n, X, $R^1$, $R^2$, $R^3$ und $R^4$ die unter B) angegebene Bedeutung besitzen,
F) $R_a$ und $R_c$ stehen für Wasserstoff, $R_b$ steht für den Rest

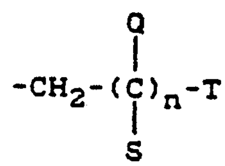

und $R_d$ für den Rest

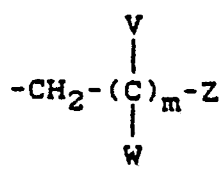

wobei
Q, S, T, V, W, Z, n und m die unter A) angegebene Bedeutung besitzen,
eine stark ausgeprägte Wirksamkeit gegen tierische Schädlinge und insbesondere gegen Spinnentiere besitzen.

Somit ergeben sich die folgenden, untereinander im Zusammenhang stehenden Strukturen $I_A$, $I_B$, $I_C$, $I_D$, $I_E$, $I_F$:

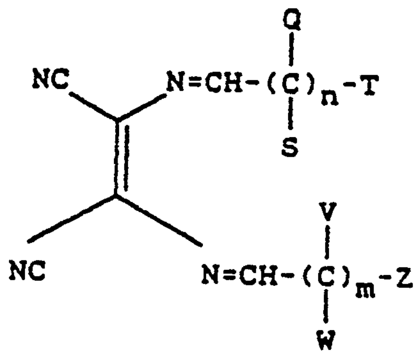

$(I_A)$

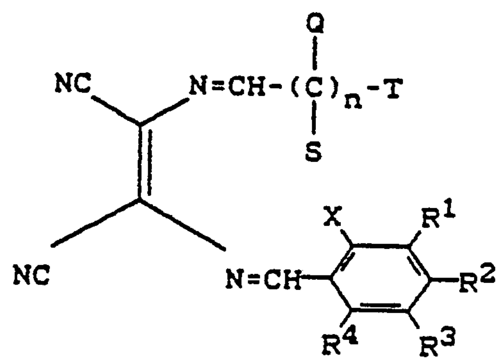

$(I_B)$

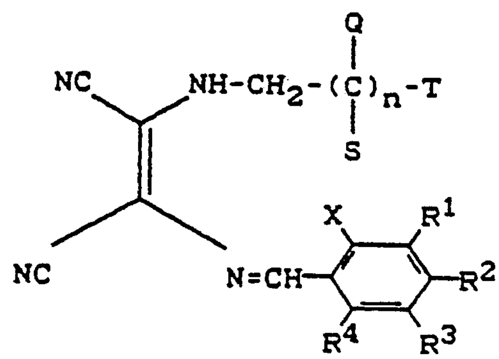

$(I_C)$

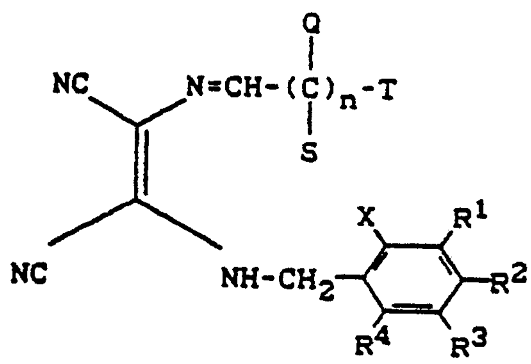

(I_D)

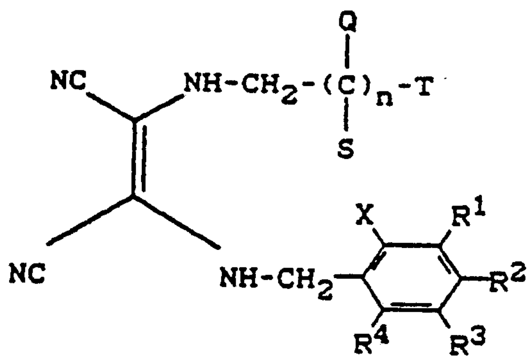

(I_E)

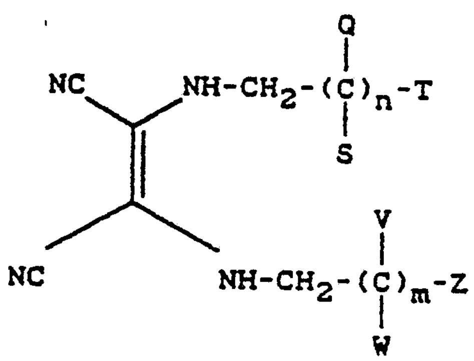

(I_F)

wobei die Reste Q, S, T, V, W, Z, X, $R^1$, $R^2$, $R^3$, $R^4$ sowie m und n die oben angegebene Bedeutung besitzen.

Allgemein ist zu sagen, daß die Verbindungen der allgemeinen Formel (I) an sich sowohl in cis-Form

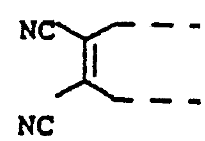

als auch in der stereoisomeren trans-Form

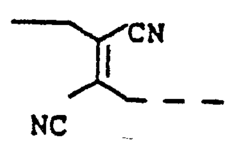

vorliegen können.

Vorzugsweise liegen die Verbindungen der allgemeinen Formel (I) in der cis-Form vor. Überraschenderweise zeigen die Verbindungen der allgemeinen Formel (I) eine stark ausgeprägte Wirksamkeit gegenüber Spinnentieren. Eine derartige Wirkung ähnlich oder gleich strukturierter Verbindungen des Standes der Technik ist bisher nicht bekannt geworden.

Man erhält die Bisazomethine der Formel $I_A$

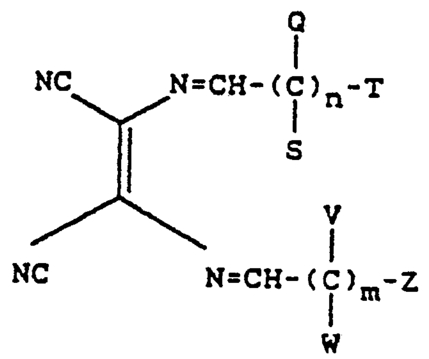

$(I_A)$

in welcher die Reste Q, S, T, V, W, Z, n und m die oben angegebene Bedeutung besitzen, indem man 2,3-Diaminomaleinsäurenitril ("DAMN") der Formel (II)

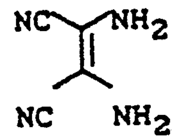

(II)

in äquimolaren Mengen in einem Verdünnungsmittel mit einem Aldehyd der Formel (III)

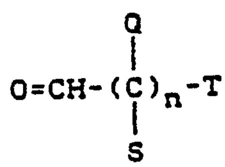

(III)

in welcher Q, S, T und n die oben angegebene Bedeutung besitzen, zum Azomethin der Formel (IV)

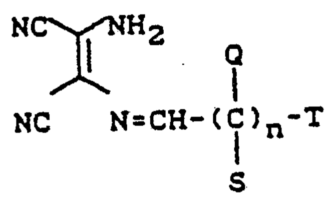

(IV)

umsetzt und dieses dann unter Zusatz eines Katalysators mit molarer Menge eines weiteren Aldehyds der Formel (V)

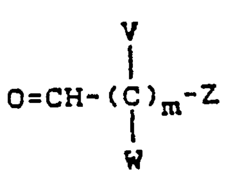

(V)

in welcher V, W, Z und m die oben angegebene Bedeutung haben, umsetzt.

Weiterhin erhält man die symmetrischen Bisazomethine der Formel (VI)

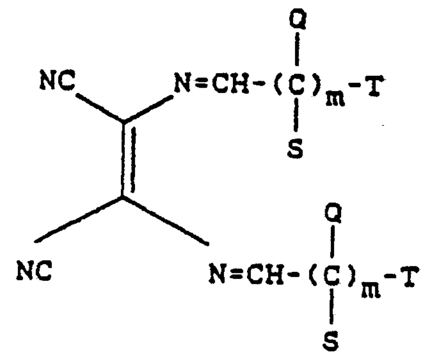

(VI)

d.h. Verbindungen der Formel $I_A$, für welche gilt: (Q = V, S = W, T = Z, m = n), indem man 1 Mol 2,3-Diaminomaleinsäurenitril (DAMN) der Formel (II)

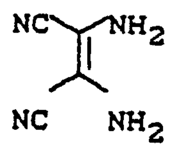

(II)

in einem Verdünnungsmittel unter Zusatz eines Katalysators mit 2 Mol eines Aldehyds der Formel (III)

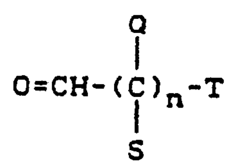

(III)

in welcher Q, S, T und n die obengenannte Bedeutung haben, umsetzt.

Weiterhin erhält man die unsymmetrischen Bisazomethine der Formel ($I_B$)

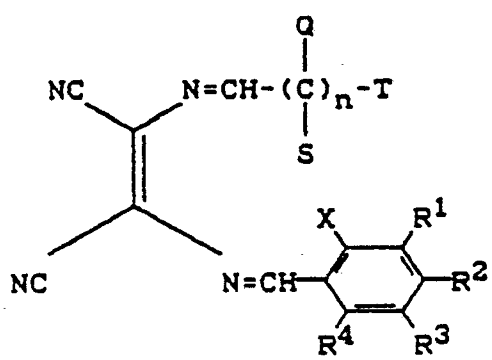

($I_B$)

in welcher Q, S, T, n, X, $R^1$, $R^2$, $R^3$ und $R^4$ die oben angegebene Bedeutung besitzen, indem man 2,3-Diaminomaleinsäurenitril der Formel (II)

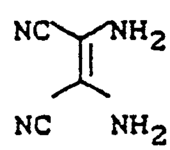

(II)

in äquimolaren Mengen in einem Verdünnungsmittel mit einem Aldehyd der Formel (III)

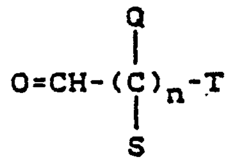

(III)

bzw. einem Aldehyd der Formel (VII)

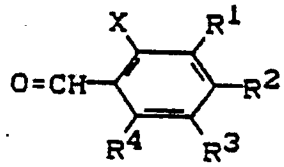

(VII)

in welcher Q, S, T, n, X, $R^1$, $R^2$, $R^3$ und $R^4$ die oben angegebene Bedeutung besitzen, zum Azomethin der Formel (IV)

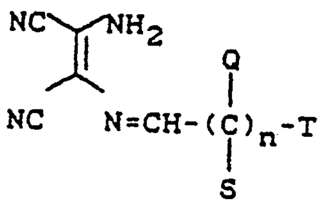

(IV)

bzw. zum Azomethin der Formel (VIII)

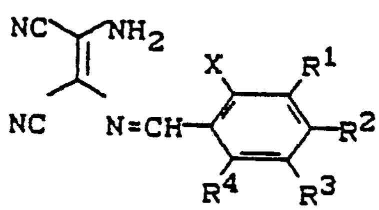

(VIII)

in welcher Q, S, T, n, X, $R^1$, $R^2$, $R^3$ und $R^4$ die oben angegebene Bedeutung haben, umsetzt und dieses dann unter Zusatz eines weiteren Aldehyds der Formel (VII)

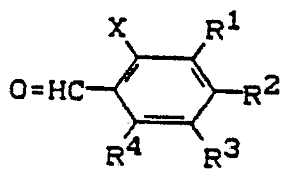

(VII)

bzw. eines weiteren Aldehyds der Formel (III)

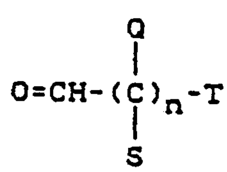

(III)

in welcher Q, S, T, n, X, $R^1$, $R^2$, $R^3$ und $R^4$ die oben angegebene Bedeutung haben, umsetzt.

Die Verbindungen der allgemeinen Formeln ($I_A$) und ($I_B$) können in den stereoisomeren cis- und/oder

trans-Formen vorliegen. Vorzugsweise liegen die Verbindungen jedoch in der cis-Form vor.

Die akarizid wirksamen Bisanile sind durch die Formeln ($I_A$) und ($I_B$) allgemein definiert.

In der Formel ($I_A$) stehen Q, S, T, V, W und Z, welche gleich oder verschieden sein können, vorzugsweise für Wasserstoff, Fluor, Chlor, Brom, Iod, Methyl, Ethyl, n-und i-Propyl, n-, i-, t-Butyl, $CF_3$, halogensubstituiertes Aryl, gegebenenfalls substituiertes Alkyl- oder Aryl-Sulfonyl.

Für n und m, welche gleich oder verschieden sein können, stehen die Zahlen 0, 1, 2, 3, 3, 5, 6 bevorzugt.

Ganz besonders bevorzugt sind Verbindungen der allgemeinen Formel ($I_A$), in denen unabhängig voneinander n und m für die Zahl 1, 2, 3, 4 stehen und Q, S, T, V, W und Z unabhängig voneinander für Wasserstoff, Fluor, Chlor, Brom, $CF_3$, Methyl, Ethyl, Phenyl, 2-Chlor-Phenyl, 2,6-Dichlorphenyl, Methylsulfonyl, halogensubstituiertes Arylsulfonyl stehen.

Außerordentlich bevorzugt sind Verbindungen der allgemeinen Formel ($I_{A'}$)

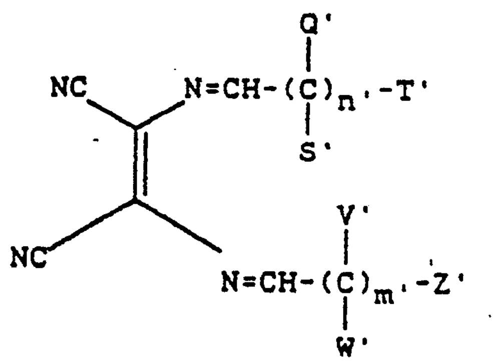

($I_{A'}$)

in der

Q′, S′ und T′ unabhängig voneinander für Fluor, Chlor, Brom, Methyl, Methylsulfonyl,

n′ für 1 oder 2,

V′, W′ und Z′ unabhängig voneinander für Wasserstoff, Chlor, Brom, Methyl, Methylsulfonyl, 2-Chlorphenyl, 2,6-Dichlorphenyl und m′ für 1, 2, 3 stehen.

Hervorragend geeignet sind Verbindungen der allgemeinen Formel ($I_{A''}$)

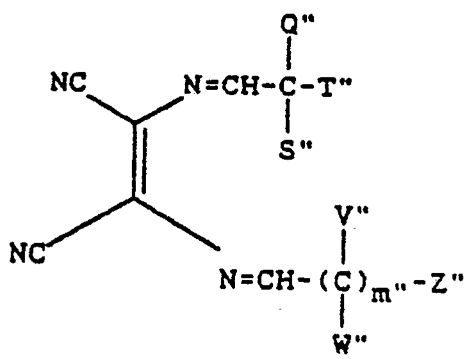

($I_{A''}$)

in welcher

Q″, S″ und T″ unabhängig voneinander für Chlor, Brom und Methyl,

V″, W″ und Z″ unabhängig voneinander für Chlor, Methyl, Methylsulfonyl und

m″ für 1 oder 2 stehen.

Im einzelnen seien außer den bei den Herstellungsbeispielen genannten Verbindungen die folgenden Bisazomethine des 2,3-Diaminomaleinsäurenitrils der Formel ($I_A$) beispielhaft genannt:

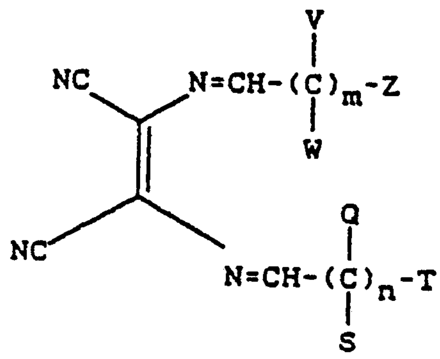

$(I_A)$

| Q | S | T | n | V | W | Z | m |
|---|---|---|---|---|---|---|---|
| H | H | H | 1 | $CH_3$ | $CH_3$ | $CH_3$ | 1 |
| $C_2H_5$ | $C_2H_5$ | H | 1 | $CH_3$ | $CH_3$ | $CH_3$ | 1 |
| H | H | $CH_3$ | 2 | $C_2H_5$ | $CH_3$ | H | 1 |
| H | H | $CH_3$ | 3 | $CH(CH_3)_2$ | $CH(CH_3)_2$ | $CH(CH_3)_2$ | 1 |
| $C_2H_5$ | $CH_3$ | H | 1 | F | F | F | 1 |
| $CH_3$ | $CH_3$ | $CH_3$ | 1 | Cl | Cl | Cl | 1 |
| $CH_3$ | $CH_3$ | $CH_3$ | 1 | Br | Br | Br | 1 |
| $C_2H_5$ | H | $C_2H_5$ | 1 | F | F | F | 1 |
| $CH_3$ | $CH_3$ | $CH_3$ | 1 | F | F | $CF_3$ | 2 |
| - | - | H | 0 | $CH_3$ | $CH_3$ | $CH_3$ | 1 |
| - | - | H | 0 | Cl | Cl | Cl | 1 |
| - | - | H | 0 | F | F | F | 1 |
| - | - | H | 0 | Br | Br | Br | 1 |
| - | - | H | 0 | F | F | $CCl_3$ | 1 |
| - | - | H | 0 | F | F | $CF_3$ | 1 |
| J | J | J | 1 | $C_2H_5$ | H | $C_2H_5$ | 1 |
| $CH_3$ | $CH_3$ | H | 1 | F | F | $OCH_3$ | 1 |
| $C_2H_5$ | $CH_3$ | $C_2H_5$ | 1 | Cl | Cl | Cl | 1 |
| - | - | $C_2H_5$ | 0 | F | F | $OCF_3$ | 1 |
| - | - | H | 0 | $CF_3$ | $CF_3$ | $CF_3$ | 1 |
| $C_2H_5$ | $CH_3$ | $CH_3$ | 1 | $CCl_3$ | $CCl_3$ | $CCl_3$ | 1 |
| - | - | H | 1 | H | H | 2-Chlor-phenyl | 1 |
| - | - | H | 1 | H | H | 2,6-Di-chlorphenyl | 1 |
| - | - | H | 1 | H | H | 2,4,6-Trichlorphenyl | 1 |

| Q | S | T | n | V | W | Z | m |
|---|---|---|---|---|---|---|---|
| - | - | H | 1 | H | H | 2-Brom-phenyl | 1 |
| - | - | H | 1 | H | H | 2-Chlor-6-fluorphenyl | 1 |
| - | - | H | 1 | H | H | 2,4-Dichlor-phenyl | 1 |
| - | - | H | 1 | H | H | 3,4-Dichlor-phenyl | 1 |
| $CH_3$ | $CH_3$ | $CH_3$ | 1 | H | H | 2,6-Dichlor-phenyl | 1 |
| $C_2H_5$ | $C_2H_5$ | $CH_3$ | 1 | H | H | 2,6-Dichlor-phenyl | 1 |
| $CH_3$ | $CH_3$ | $CH_3$ | 1 | H | H | Phenyl | 1 |
| $CH_3$ | $CH_3$ | $CH_3$ | 1 | H | H | 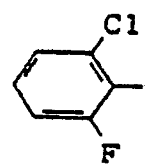 | 1 |
| 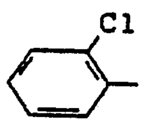 | H | H | 1 | H | H | 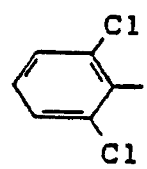 | 1 |
| 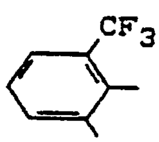 | H | H | 1 | H | H | 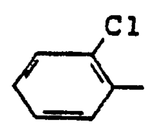 | 1 |
| 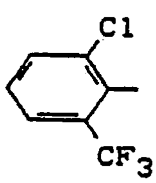 | H | H | 1 | H | H | 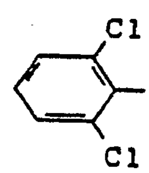 | 1 |

| Q | S | T | n | V | W | Z | m |
|---|---|---|---|---|---|---|---|
| 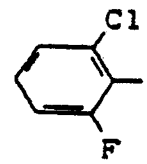 | H | H | 1 | H | H | 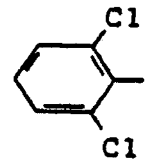 | 1 |
| 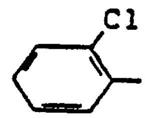 | H | H | 1 | H | H | 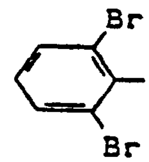 | 1 |
| 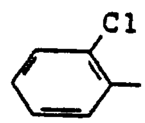 | H | H | 1 | H | H | 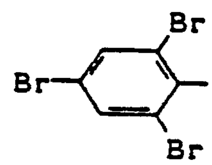 | 1 |
| 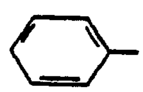 | H | H | 1 | H | H | 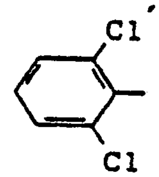 | 1 |
| 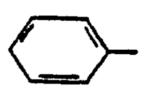 | H | H | 1 | H | H | 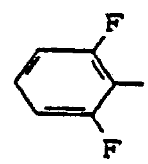 | 1 |
| Cl | Cl | Cl | 1 | H | H | 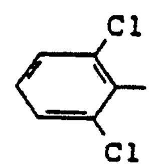 | 1 |
| Br | Br | Br | 1 | H | H | 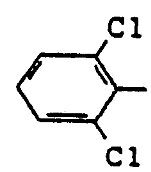 | 1 |
| F | F | F | 1 | H | H | 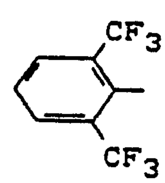 | 1 |

In der Formel ($I_B$) stehen Q, S und T, welche gleich oder verschieden sein können, vorzugsweise für Wasserstoff, Fluor, Chlor, Brom, Iod, Methyl, Ethyl, n- und i-Propyl, n-, i-, t-Butyl, $CF_3$, halogensubstituiertes Aryl, gegebenenfalls substituiertes Alkyl- und Arylsulfonyl; für n stehen vorzugsweise die Zahlen 0, 1, 2, 3, 4, 5, 6, X steht bevorzugt für Fluor, Chlor, Brom, Iod, $CF_3$ oder CN.

Bei den Resten $R^1$, $R^2$, $R^3$ und $R^4$, welche ebenfalls gleich oder verschieden sein können, kommen als Substituenten für Alkyl, Alkoxy, Dialkylamino, Alkylthio, Alkylthionyl, Alkylsulfonyl vorzugsweise infrage:

Halogen, insbesondere Fluor, Chlor und Brom sowie OH, $NH_2$.

Bevorzugt sind Verbindungen der Formel ($I_B$), in welcher
X und $R^4$ gleich oder verschieden sein können und für Wasserstoff, Fluor, Chlor, Brom, Iod, $CF_3$ oder CN stehen,
$R^1$, $R^2$ und $R^3$ gleich oder verschieden sein können und für Wasserstoff, Alkyl($C_1$-$C_4$), Halogenalkyl($C_1$-$C_4$), Alkoxy($C_1$-$C_4$), Halogenalkoxy($C_1$-$C_4$), Halogen, CN, $NO_2$, Dialkyl($C_1$-$C_4$)amino, Alkoxy($C_1$-$C_4$)carbonyl, Alkyl($C_1$-$C_4$)thio, Alkyl($C_1$-$C_4$)thionyl, Dihalogenalkyl($C_1$-$C_4$)amino, Alkyl($C_1$-$C_4$)sulfonyl, OH, SH, $NH_2$ stehen.

Besonders bevorzugt sind Verbindungen der allgemeinen Formel ($I_B'$)

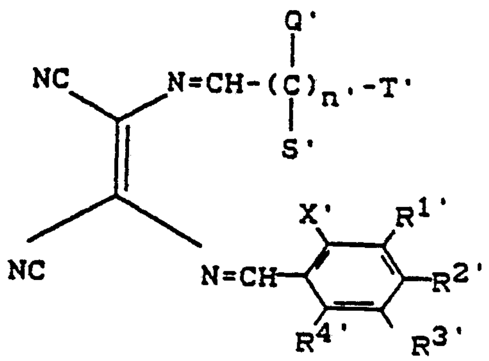

($I_{B'}$)

in der
$Q'$, $S'$ und $T'$, welche gleich oder verschieden sein können, für Wasserstoff, Fluor, Chlor, Brom, $CF_3$, Methyl, Ethyl, 2-Chlorphenyl, 2,6-Dichlorphenyl, Methylsulfonyl, halogensubstituiertes Arylsulfonyl,
$n'$ für die Zahlen 0, 1, 2, 3, 4,
$X'$ für Fluor, Chlor, Brom, Iod oder $CF_3$,
$R^1$, $R^2$, $R^3$ und $R^4$ für Wasserstoff, Fluor, Chlor, Brom, $CF_3$, CN, Alkyl($C_1$-$C_4$), Halogenalkyl($C_1$-$C_4$), Alkoxy($C_1$-$C_4$), Halogenalkoxy($C_1$-$C_4$), $NO_2$, Alkoxy($C_1$-$C_4$)carbonyl, Alkyl($C_1$-$C_4$)thio, Alkyl($C_1$-$C_4$)thionyl, Alkyl($C_1$-$C_4$)sulfonyl, OH oder SH stehen.

Ganz besonders bevorzugt sind Verbindungen der allgemeinen Formel ($I_B''$)

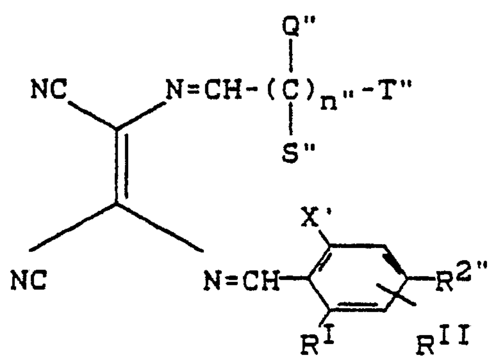

($I_{B''}$)

in der
$Q''$, $S''$ und $T''$ gleich oder verschieden sein können und für Wasserstoff, Fluor, Chlor, Brom, Methyl, Methylsulfonyl, 2-Chlorphenyl, 2,6-Dichlorphenyl,
$n''$ für 1 oder 2,
$X'$ und $R^I$ gleich oder verschieden sein können und für Fluor, Chlor, Brom, Iod oder $CF_3$ stehen
und
$R^{II}$ für Wasserstoff, Chlor, Brom, Iod, Cyan, Nitro, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Pentafluorethyl, Trifluorethyl, Trifluormethylsulfon, Trifluormethoxy, Trichlormethoxy, Pentafluorethoxy, Dimethylamino, Diethylamino,, Di-$\beta$-chlorethylamino, Di-$\beta$-hydroxyethylamino und
$R^2$ für Wasserstoff, Alkyl($C_1$-$C_4$), Halogenalkyl($C_1$-$C_4$), Alkoxy($C_1$-$C_4$), Halogenalkoxy($C_1$-$C_4$), Halogen, CN, $NO_2$, Dialkyl($C_1$-$C_4$)amino, Alkoxy($C_1$-$C_4$)carbonyl, Alkyl($C_1$-$C_4$)thio, Alkyl($C_1$-$C_4$)thionyl, Dihalogenalkyl($C_1$-

$C_4$)amino, Alkyl($C_1$-$C_4$)sulfonyl, OH, SH, $NH_2$ steht.

Ganz besonders bevorzugt sind Verbindungen der allgemeinen Formel ($I_{B'''}$)

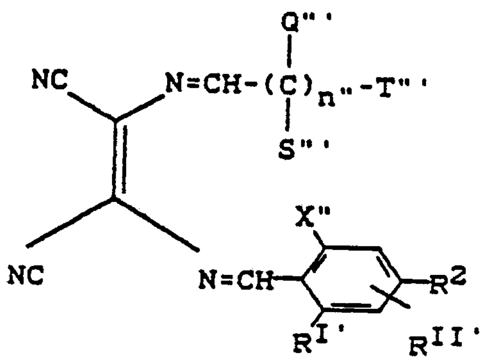

($I_{B'''}$)

in der

Q''', S''' und T''' unabhängig voneinander für Wasserstoff, Fluor, Chlor, Brom, Methyl,
n'' für 1 oder 2,
X'' und $R^I$ gleich oder verschieden sein können und für Chlor oder Brom stehen
und
$R^{II}$ für Chlor, Brom, Iod, Wasserstoff, Cyan, Nitro, Methyl, Ethyl, n- und i-Propyl, Methoxy, Ethoxy, Methoxycarbonyl, Ethoxycarbonyl, Trifluormethyl, Trichlormethyl, Dimethylamino, Diethylamino, Trifluormethylsulfon und
$R^2$ für Wasserstoff, Alkyl($C_1$-$C_4$), Halogenalkyl($C_1$-$C_4$), Alkoxy($C_1$-$C_4$), Halogenalkoxy($C_1$-$C_4$), Halogen, CN, $NO_2$, Dialkyl($C_1$-$C_4$)amino, Alkoxy($C_1$-$C_4$)carbonyl, Alkyl($C_1$-$C_4$)thio, Alkyl($C_1$-$C_4$)thionyl, Dihalogenalkyl($C_1$-$C_4$)amino, Alkyl($C_1$-$C_4$)sulfonyl, OH, SH, $NH_2$ steht.

Von ganz besonderem Interesse sind Verbindungen der allgemeinen Formel ($I_{B''''}$)

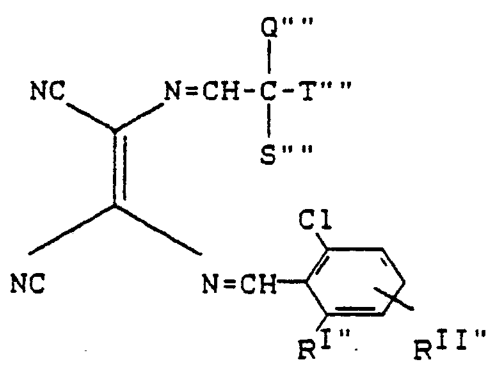

($I_{B''''}$)

in der

Q'''', S'''' und T'''' unabhängig voneinander für Fluor, Chlor, Brom, Methyl,
$R^{I''}$ für Chlor oder Brom steht und
$R^{II}$ für Wasserstoff, Chlor, Methyl, Trichlormethyl, Trifluormethyl, Methoxy, Trichlormethoxy oder Trifluormethoxy stehen.

Verwendet man beispielsweise 2,3-Diaminomaleinsäurenitril (DAMN) und 2,2-Dimethyl-propanol als Ausgangsprodukte, so kann das entsprechende Bisazomethin wie folgt dargestellt werden.

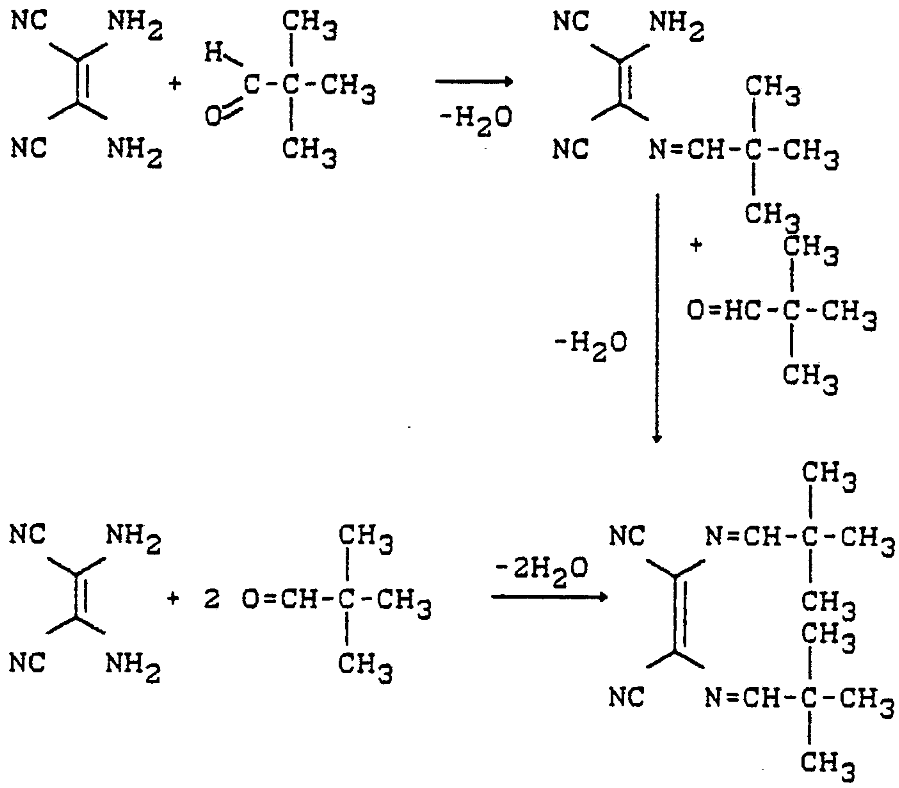


Die zur Durchführung des Verfahrens zur Herstellung der Bisazomethine ($I_A$) und ($I_B$) als Ausgangsstoffe benötigten Aldehyde sind durch die Formeln (III) bzw. (V) allgemein definiert. Es handelt sich im wesentlichen um bekannte Verbindungen. Siehe dazu z.B. O. Bayer, in Houben-Weyl, Bd. VII/1, S. 16-36 (1954).

Die weitere Ausgangsverbindung 2,3-Diaminomaleinsäurenitril ist ebenfalls literaturbekannt und im Handel erhältlich.

Als Verdünnungsmittel zur Durchführung des Verfahrens zur Herstellung der erfindungsgemäß verwendbaren Verbindungen der Formeln ($I_A$) und ($I_B$) bzw. (IV) und (VIII) kommen vorzugsweise polare organische Lösungsmittel in Frage, wie z.B. Alkohole (insbesondere Methanol, Ethanol, Propanol (n und i)), Dimethylformamid (DMF), Dimethylsulfoxid (DMSO), Hexamethylphosphorsäuretriamid (HMPT), Acetonitril.

Die Reaktionstemperatur liegt im allgemeinen im Bereich zwischen ca. 0° C bis maximal zum Siedepunkt des jeweiligen Lösungsmittels, insbesondere wird die Reaktion bei Temperaturen von ca. 20 bis ca. 100° C durchgeführt.

Die Umsetzung wird vorzugsweise bei Normaldruck durchgeführt.

Als Katalysator für die Umsetzung der Verbindungen der Formel (IV) bzw. (VIII) mit molaren Mengen des Aldehyds der Formeln (III) und (V) wird vorzugsweise eingesetzt: anorganische oder organische Säuren oder Derivate. Beispielhaft seien genannt: Chlorwasserstoffsäure, Bromwasserstoffsäure, Jodwasserstoffsäure, Schwefelsäure, Bortrifluorid, Tetrafluorborsäure, Perchlorsäure, Methansulfonsäure, Trifluormethansulfonsäure, p-Toluolsulfonsäure, Phosphorsäure, Phosphorpentoxid, Ameisensäure, Essigsäure, Propionsäure, Chlorwasserstoff, Bromwasserstoff.

Hierbei wird 0,001 bis 2 Mol Katalysator pro Mol Aldehyd der Formel (V) eingesetzt, vorzugsweise 0,01 bis 1 Mol Katalysator pro Mol Aldehyd der Formel (V).

Für die direkte Umsetzung von 2,3-Diaminomaleinsäurenitril mit 2 Mol Aldehyd der Formel (III) zum Erhalt der symmetrischen Bisanile der Formel (VI) werden vorzugsweise die obengenannten Katalysatoren in den obengenannten Mengenverhältnissen eingesetzt.

Die Verbindungen der Formel (VIII)

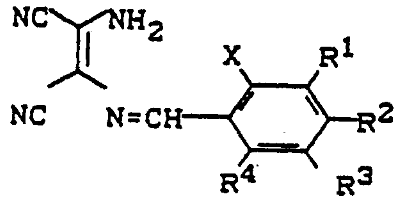

(VIII)

worin X, $R^1$, $R^2$, $R^3$ und $R^4$ die oben angegebene Bedeutung besitzen, sind Gegenstand einer älteren im Hinblick auf die vorliegende Anmeldung nicht vorveröffentlichten Patentanmeldung.

Im einzelnen seien außer den bei den Herstellungsbeispielen genannten Verbindungen die folgenden Bisazomethine des 2,3-Diaminomaleinsäurenitrils der Formel ($I_B$) beispielhaft genannt:

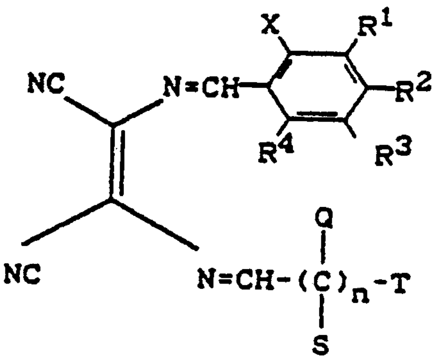

$(I_B)$

| Q | S | T | n | X | $R^1$ | $R^2$ | $R^3$ | $R^4$ |
|---|---|---|---|---|---|---|---|---|
| H | H | H | 1 | Cl | H | H | H | Cl |
| - | - | H | 0 | Cl | H | H | H | Cl |
| H | H | $CH_3$ | 1 | Cl | H | H | H | Cl |
| $CH_3$ | $CH_3$ | $CH_3$ | 1 | Cl | H | H | H | Cl |
| $C_2H_5$ | H | $C_2H_5$ | 1 | Cl | H | H | H | Cl |
| $C_2H_5$ | $CH_3$ | $CH_3$ | $C_2H_5$ | Cl | H | H | H | Cl |
| - | - | H | 0 | Cl | H | Cl | H | Cl |
| - | - | H | 0 | Br | H | H | H | Cl |
| - | - | H | 0 | F | H | H | H | Cl |
| - | - | H | 0 | Br | H | H | H | Br |
| $CH_3$ | H | $CH_3$ | 1 | Cl | H | H | H | Cl |
| $CH_3$ | $CH_3$ | $CH_3$ | 1 | F | H | H | H | F |
| $CH_3$ | $CH_3$ | $CH_3$ | 1 | $CF_3$ | H | H | H | $CF_3$ |
| Cl | Cl | Cl | 1 | Cl | H | H | H | Cl |
| $CH_3$ | $CH_3$ | $CH_3$ | 1 | Br | H | H | H | Cl |
| $CH_3$ | $CH_3$ | $CH_3$ | 1 | Cl | H | F | H | Cl |
| - | - | H | 0 | Cl | H | F | H | Cl |
| - | - | H | 0 | Br | H | Br | H | Br |
| F | F | F | 1 | Cl | H | H | H | Cl |
| - | - | H | 0 | F | $OCF_3$ | F | F | F |
| $CH_3$ | $CH_3$ | $CH_3$ | 1 | F | $OCF_3$ | F | F | F |

| Q | S | T | n | X | $R^1$ | $R^2$ | $R^3$ | $R^4$ |
|---|---|---|---|---|---|---|---|---|
| Cl | Cl | Cl | 1 | Cl | H | H | H | Cl |
| Br | Br | Br | 1 | Cl | H | H | H | Cl |
| $CF_3$ | F | F | 1 | Cl | H | H | H | Cl |
| Cl | Cl | Cl | 1 | Cl | H | H | H | F |
| Cl | Cl | Cl | 1 | Br | H | H | H | Br |
| - | - | H | 0 | H | $CF_3$ | H | H | H |
| - | - | H | 0 | $CF_3$ | - | - | - | $CF_3$ |
| $CH_3$ | $C_2H_5$ | $C_2H_5$ | 1 | Cl | H | F | H | Cl |
| $CH_3$ | Cl | Cl | 1 | Cl | H | H | H | Cl |
| F | F | F | 1 | Br | H | H | H | Br |
| F | F | F | 1 | Cl | H | Cl | H | Cl |
| - | - | H | 0 | H | Cl | Cl | H | H |
| 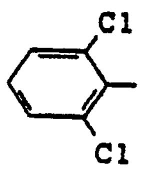 | H | H | 1 | Cl | H | H | H | Cl |
| 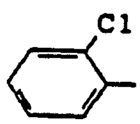 | H | H | 1 | Cl | H | H | H | Cl |
| 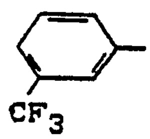 | H | H | 1 | Cl | H | H | H | Cl |

| Q | S | T | n | X | $R^1$ | $R^2$ | $R^3$ | $R^4$ |
|---|---|---|---|---|---|---|---|---|
| 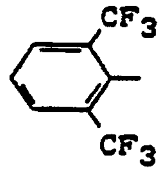 | H | H | 1 | Cl | H | H | H | Cl |
| 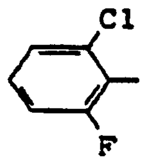 | H | H | 1 | Cl | H | H | H | Cl |
| 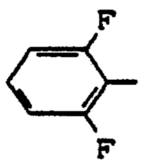 | H | H | 1 | Cl | H | H | H | Cl |
| 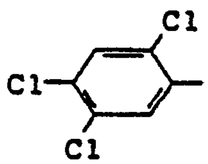 | H | H | 1 | Cl | H | H | H | Cl |
| 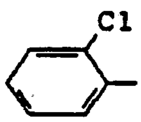 | H | H | 1 | Br | H | H | H | Br |

Man erhält die neuen Amino-imino-Derivate des 2,3-Diaminomaleinsäurenitrils der Formel $I_C$

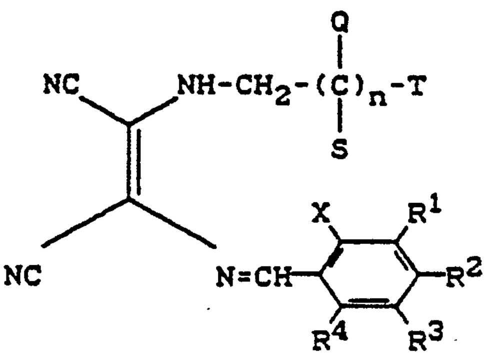

$(I_C)$.

in welcher
Q, S, T, n, X, $R^1$, $R^2$, $R^3$ und $R^4$ die unter B) angegebene Bedeutung haben,
wenn man Amino-Verbindungen der allgemeinen Formel (IX)

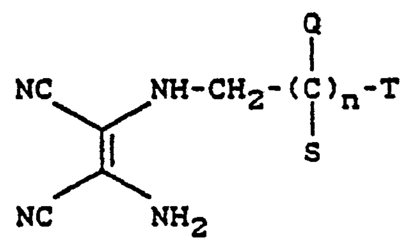

(IX)

in welcher
Q, S, T und in die unter A) angegebene Bedeutung haben, in äquimolarer Menge in einem geeigneten Verdünnungsmittel mit einem Aldehyd der Formel (VII)

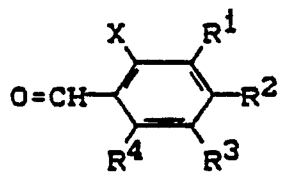

(VII)

in welcher
X, $R^1$, $R^2$, $R^3$ und $R^4$ die unter B) angegebene Bedeutung besitzen, umsetzt.

Überraschenderweise zeigen die Verbindungen der Formel ($I_C$) eine akarizide Wirksamkeit. Eine derartige Wirkung ist von der Stoffklasse der Amino-imino-Verbindungen des Diaminomaleinsäurenitrils bisher noch nicht bekannt geworden. Die erfindungsgemäßen Verbindungen stellen somit eine Bereicherung des Standes der Technik dar.

Die Verbindungen der allgemeinen Formel ($I_C$) können in den stereoisomeren cis- und/oder trans-Formen vorliegen, vorzugsweise liegen die Verbindungen jedoch in der cis-Form vor.

In der Formel ($I_C$) steht X vorzugsweise für Wasserstoff, Fluor, Chlor, Brom, Iod, $CF_3$ oder CN.

Bei den Resten $R^1$, $R^2$, $R^3$ und $R^4$ kommen als Substituenten für Alkyl, Alkoxy, Dialkylamino, Alkylthio, Alkylthionyl, Alkylsulfonyl vorzugsweise infrage: Halogen, insbesondere Fluor, Chlor und Brom sowie OH, $NH_2$.

Q, S und T stehen vorzugsweise unabhängig voneinander für Wasserstoff, Fluor, Chlor, Brom, Iod, Methyl, Ethyl, n- und i-Propyl, n-, i-, t-Butyl, $CF_3$, halogensubstituiertes Aryl, gegebenenfalls substituiertes Alkyl- und Arylsulfonyl.

Für n sind die Zahlen 0, 1, 2, 3, 4, 5, 6 bevorzugt.

Ganz besonders bevorzugt sind Verbindungen der allgemeinen Formel ($I_C$), in der unabhängig voneinander n für die Zahl 0, 1, 2, 3 oder 4 steht,
Q, S und T unabhängig voneinander für Wasserstoff, Fluor, Chlor, Brom, $CF_3$, Methyl, Ethyl, Phenyl, 2-Chlorphenyl, 2,6-Dichlorphenyl, Methylsulfonyl, halogensubstituiertes Arylsulfonyl,
X für Wasserstoff, Fluor, Chlor, Brom, CN und $CF_3$ und die Reste $R^1$, $R^2$, $R^3$ und $R^4$ unabhängig voneinander für Wasserstoff, Alkyl($C_1$-$C_4$), Halogenalkyl($C_1$-$C_4$), Alkoxy($C_1$-$C_4$), Halogenalkoxy($C_1$-$C_4$), Dialkyl($C_1$-$C_4$)amino, Alkoxy($C_1$-$C_4$)carbonyl, Alkyl($C_1$-$C_4$)thio, Alkyl($C_1$-$C_4$)thionyl, Dihalogenalkyl($C_1$-$C_4$)-amino, Alkyl($C_1$-$C_4$)sulfonyl, OH, $NH_2$ stehen.

Außerordentlich bevorzugt sind Verbindungen der allgemeinen Formel ($I_C'$)

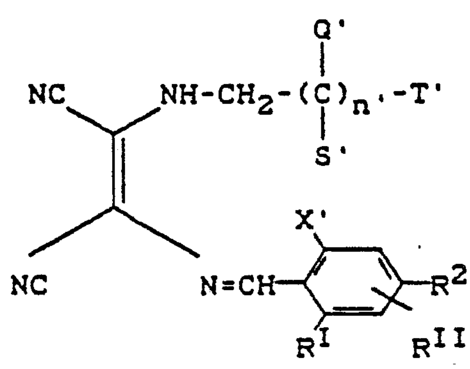

($I_{C'}$)

in der
$Q'$, $S'$ und $T'$ gleich oder verschieden sein können und für Wasserstoff, Fluor, Chlor, Brom, Methyl, Methylsulfonyl, 2-Chlorphenyl, 2,6-Dichlorphenyl,
$n'$ für 1 oder 2,
$X'$ und $R^{I}$ gleich oder verschieden sein können und für Fluor, Chlor, Brom, Iod oder $CF_3$ stehen
und
$R^{II}$ für Wasserstoff, Chlor, Brom, Iod, Cyan, Nitro, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Pentafluorethyl, Trifluorethyl, Trifluormethylsulfon, Trifluormethoxy, Trichlormethoxy, Pentafluorethoxy, Dimethylamino, Diethylamino, Di-β-chlorethylamino, Di-β-hydroxyethylamino und
$R^2$ für Wasserstoff, Alkyl($C_1$-$C_4$), Halogenalkyl($C_1$-$C_4$), Alkoxy($C_1$-$C_4$), Halogenalkoxy($C_1$-$C_4$), Halogen, CN, $NO_2$, Dialkyl($C_1$-$C_4$)amino, Alkoxy($C_1$-$C_4$)carbonyl, Alkyl($C_1$-$C_4$)thio, Alkyl($C_1$-$C_4$)thionyl, Dihalogenalkyl($C_1$-$C_4$)amino, Alkyl($C_1$-$C_4$)sulfonyl, OH, SH, $NH_2$ stehen.

Ganz besonders bevorzugt sind Verbindungen der allgemeinen Formel ($I_C''$)

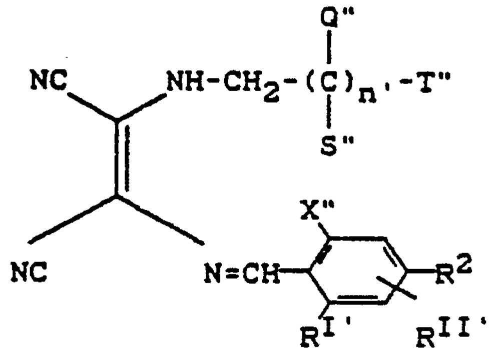

($I_{C''}$)

in der
$Q''$, $S''$ und $T''$ unabhängig voneinander für Wasserstoff, Fluor, Chlor, Brom, Methyl,
$n'$ für 1 oder 2,
$X''$ und $R^{I}$ gleich oder verschieden sein können und für Chlor oder Brom stehen
und
$R^{II}$ für Chlor, Brom, Iod, Wasserstoff, Cyan, Nitro, Methyl, Ethyl, n- und i-Propyl, Methoxy, Ethoxy, Methoxycarbonyl, Ethoxycarbonyl, Trifluormethyl, Trichlormethyl, Dimethylamino, Diethylamino, Trifluormethylsulfon und
$R^2$ für Wasserstoff, Alkyl($C_1$-$C_4$), Halogenalkyl($C_1$-$C_4$), Alkoxy($C_1$-$C_4$), Halogenalkoxy($C_1$-$C_4$), Halogen, CN, $NO_2$, Dialkyl($C_1$-$C_4$)amino, Alkoxy($C_1$-$C_4$)carbonyl, Alkyl($C_1$-$C_4$)thio, Alkyl($C_1$-$C_4$)thionyl, Dihalogenalkyl($C_1$-$C_4$)amino, Alkyl($C_1$-$C_4$) sulfonyl, OH, SH, $NH_2$ stehen.

Von ganz besonderem Interesse sind Verbindungen der allgemeinen Formel ($I_C'''$)

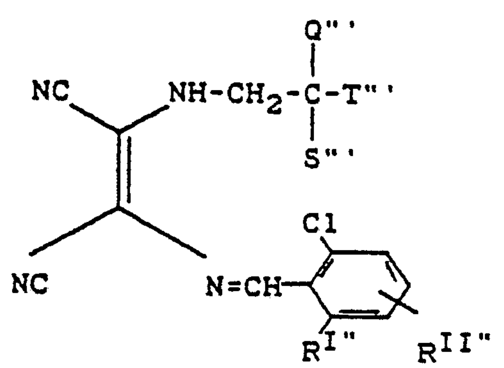

($I_{C'''}$)

in der
$Q'''$, $S'''$ und $T'''$ unabhängig voneinander für Fluor, Chlor, Brom oder Methyl,
$R^{I}$ „ für Chlor oder Brom steht und
$R^{II}$ für Wasserstoff, Chlor, Methyl, Trichlormethyl, Trifluormethyl, Methoxy, Trichlormethoxy oder Trifluormethoxy stehen.

Verwendet man beispielsweise die N-2,2-Dimethyl-propyl-Verbindung des 2,3-Diaminomaleinsäurenitrils und den 2,6-Dichlorbenzaldehyd als Ausgangsprodukte, so kann das Herstellungsverfahren zum Erhalt der Verbindung $I_C$ wie folt dargestellt werden:

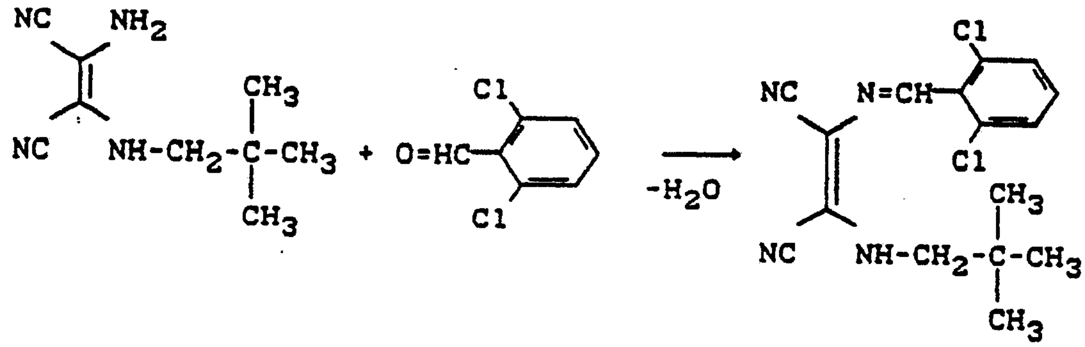


Die zur Durchführung des erfindungsgemäßen Verfahrens als Ausgangsstoffe benötigten Aldehyde sind durch die Formel (V) allgemein definiert. Es handelt sich im wesentlichen um bekannte Verbindungen. Siehe dazu z.B. O. Bayer, in Houben-Weyl, Bd. VII/1, S. 16-36 (1954).

Als Verdünnungsmittel zur Durchführung des erfindungsgemäßen Verfahrens kommen vorzugsweise polare organische Lösungsmittel in Frage, wie z. B. Alkohole (insbesondere Methanol, Ethanol, Propanol (n und i)), Dimethylformamid (DMF), Dimethylsulfoxid (DMSO), Hexamethylphosphorsäuretriamid (HMPT), Acetonitril.

Die Reaktionstemperatur liegt im allgemeinen im Bereich zwischen ca. 0 °C bis maximal zum Siedepunkt des jeweiligen Lösungsmittels, insbesondere wird die Reaktion bei Temperaturen von ca. 20 bis ca. 100 °C durchgeführt.

Die Umsetzung wird vorzugsweise bei Normaldruck durchgeführt.

Zur Durchführung des erfindungsgemäßen Verfahrens setzt man bevorzugt äquimolare Mengen der Reaktionspartner ($I_C$) und (V) miteinander um. Es kann aber auch ein leichter Überschuß eines der beiden Reaktionspartner angewendet werden.

In einer bevorzugten Ausführungsform gibt man bei Raumtemperatur die beiden Reaktionspartner im Verdünnungsmittel zusammen und erhitzt anschließend zum Rückfluß.

Die Aufarbeitung erfolgt nach üblichen Methoden, nach dem Abkühlen wird das Reaktionsprodukt vorzugsweise abgesaugt und nach an sich bekannten Methoden aufgearbeitet.

Im einzelnen seien außer den bei den Herstellungsbeispielen genannten Verbindungen die folgenden Bisazomethine des 2,3-Diaminomaleinsäurenitrils der Formel ($I_C$) beispielhaft genannt:

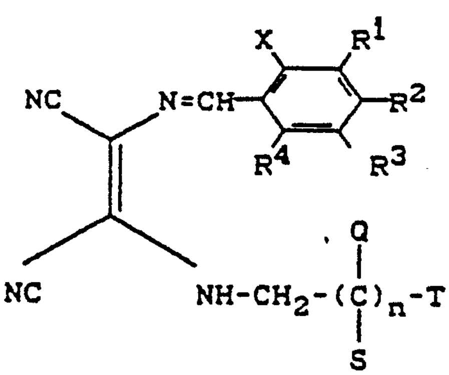

($I_C$)

| Q | S | T | n | X | $R^1$ | $R^2$ | $R^3$ | $R^4$ |
|---|---|---|---|---|---|---|---|---|
| - | - | H | 0 | Cl | H | H | H | Cl |
| - | - | H | 0 | F | H | H | H | Cl |
| - | - | H | 0 | F | H | H | H | F |
| - | - | H | 0 | Br | H | H | H | Br |
| - | - | H | 0 | Cl | H | Cl | H | Cl |
| $CH_3$ | $CH_3$ | $CH_3$ | 1 | Cl | H | H | H | Cl |
| $CH_3$ | $CH_3$ | $CH_3$ | 1 | Br | H | H | H | Br |
| $C_2H_5$ | H | $C_2H_5$ | 1 | Cl | H | H | H | Cl |
| $C_2H_5$ | $CH_3$ | $C_2H_5$ | 1 | Cl | H | H | H | Cl |
| $CH_3$ | $CH_3$ | $CH_3$ | 1 | H | $CF_3$ | H | H | H |
| $CH_3$ | $CH_3$ | $CH_3$ | 1 | H | Cl | Cl | H | H |
| H | H | H | 1 | Cl | H | H | H | Cl |
| H | H | H | 1 | H | Cl | Cl | H | H |
| H | H | H | 1 | Cl | H | Cl | H | H |
| H | H | H | 1 | Br | H | H | H | Br |
| Cl | Cl | Cl | 1 | Cl | H | H | H | Cl |
| Br | Br | Br | 1 | Cl | H | H | H | Cl |
| H | H | $-SO_2CH_3$ | 1 | Cl | H | H | H | Cl |
| F | F | F | 1 | Cl | H | H | H | Cl |
| Cl | Cl | Cl | 1 | $CF_3$ | H | H | H | $CF_3$ |
| Cl | Cl | Cl | 1 | Br | H | H | H | Br |
| Br | Br | Br | 1 | Cl | H | H | H | F |
| Br | Br | Br | 1 | F | H | H | H | F |
| $CH_3$ | $CH_3$ | $CH_3$ | 1 | $CF_3$ | H | H | H | $CF_3$ |
| Cl | Cl | Cl | 1 | Cl | H | Cl | H | Cl |
| Br | Br | Br | 1 | Cl | H | Cl | H | Cl |
| F | F | F | 1 | Cl | H | Cl | H | Cl |
| Cl | Cl | Cl | 1 | F | $OCF_3$ | F | F | F |

| Q | S | T | n | X | $R^1$ | $R^2$ | $R^3$ | $R^4$ |
|---|---|---|---|---|---|---|---|---|
| 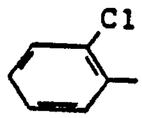 | H | H | 1 | Cl | H | H | H | Cl |
| 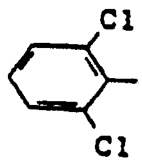 | H | H | 1 | Cl | H | H | H | Cl |
| 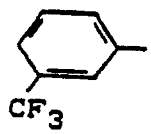 | H | H | 1 | Cl | H | H | H | Cl |
| 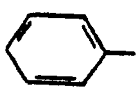 | H | H | 1 | Cl | H | H | H | Cl |
| 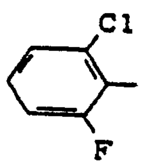 | H | H | 1 | Cl | H | H | H | Cl |
| 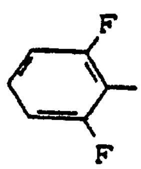 | H | H | 1 | Cl | H | H | H | Cl |
| 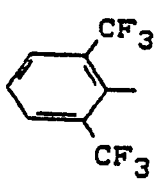 | H | H | 1 | Cl | H | H | H | Cl |
| 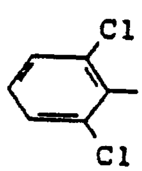 | H | H | 1 | Cl | H | H | H | H |
| 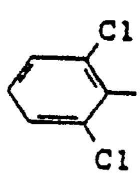 | H | H | 1 | H | Cl | Cl | H | H |

Man erhält die neuen Amino-imino-Derivate des 2,3-Diaminomaleinsäurenitrils der Formel ($I_D$)

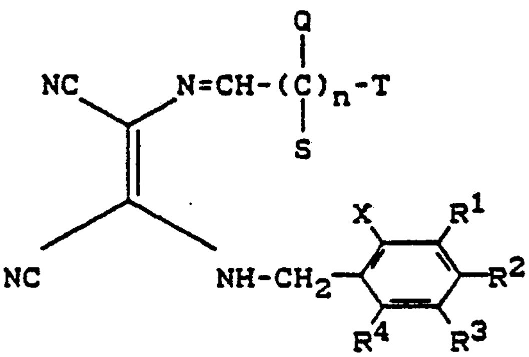

$(I_D)$

in welcher
Q, S, T, n, X, $R^1$, $R^2$, $R^3$ und $R^4$ die unter B) angegebene Bedeutung haben, wenn man Amino-Verbindungen der allgemeinen Formel (X)

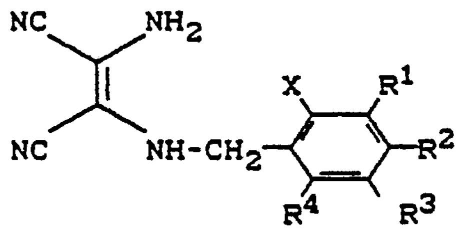

(X)

in welcher
X, $R^1$, $R^2$, $R^3$ und $R^4$ die unter B) angegebene Bedeutung haben, in äquimolarer Menge in einem geeigneten Verdünnungsmittel mit einem Aldehyd der Formel (III)

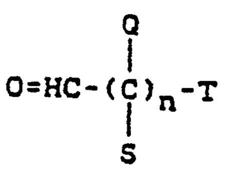

(III)

in welcher
Q, S, T und n die unter A) angegebene Bedeutung besitzen, umsetzt.

Überraschenderweise zeigen die Verbindungen der Formel $(I_D)$ eine akarizide Wirksamkeit. Eine derartige Wirkung ist von der Stoffklasse der Amino-imino-Verbindungen des Diaminomaleinsäurenitrils bisher noch nicht bekannt geworden. Die erfindungsgemäßen Verbindungen stellen somit eine Bereicherung des Standes der Technik dar.

Die Verbindungen der allgemeinen Formel $(I_D)$ können in den stereoisomeren cis- und/oder trans-Formen vorliegen, vorzugsweise liegen die Verbindungen jedoch in der cis-Form vor.

In der Formel $(I_D)$ stehen X vorzugsweise für Wasserstoff, Fluor, Chlor, Brom, Iod, $CF_3$ oder CN.

Bei den Resten $R^1$, $R^2$, $R^3$ und $R^4$ kommen als Substituenten für Alkyl, Alkoxy, Dialkylamino, Alkylthio, Alkylthionyl, Alkylsulfonyl vorzugsweise infrage: Halogen, insbesondere Fluor, Chlor und Brom sowie OH, $NH_2$.

Q, S und T stehen vorzugsweise unabhängig voneinander für Wasserstoff, Fluor, Chlor, Brom, Iod, Methyl, Ethyl, n- und i-Propyl, n-, i-, t-Butyl, $CF_3$, halogensubstituiertes Aryl, gegebenenfalls substituiertes Alkyl- und Arylsulfonyl.

Für n sind die Zahlen 0, 1, 2, 3, 4, 5, 6 bevorzugt.

Ganz besonders bevorzugt sind Verbindungen der allgemeinen Formel $(I_D)$, in der unabhängig voneinander n für die Zahl 0, 1, 2, 3 oder 4 steht,
Q, S, und T unabhängig voneinander für Wasserstoff, Fluor, Chlor, Brom, $CF_3$, Methyl, Ethyl, Phenyl, 2-Chlorphenyl, 2,6-Dichlorphenyl, Methylsulfonyl, halogensubstituiertes Arylsulfonyl,
X für Wasserstoff, Fluor, Chlor, Brom, CN und $CF_3$ und die Reste $R^1$, $R^2$, $R^3$ und $R^4$ unabhängig

voneinander für Wasserstoff, Alkyl($C_1$-$C_4$), Halogenalkyl($C_1$-$C_4$), Alkoxy($C_1$-$C_4$), Halogenalkoxy($C_1$-$C_4$), Dialkyl($C_1$-$C_4$)amino, Alkoxy($C_1$-$C_4$)carbonyl, Alkyl($C_1$-$C_4$)thio, Alkyl($C_1$-$C_4$)thionyl, Dihalogenalkyl($C_1$-$C_4$)-amino, Alkyl($C_1$-$C_4$)sulfonyl, OH, $NH_2$ stehen.

Außerordentlich bevorzugt sind Verbindungen der allgemeinen Formel ($I_D'$)

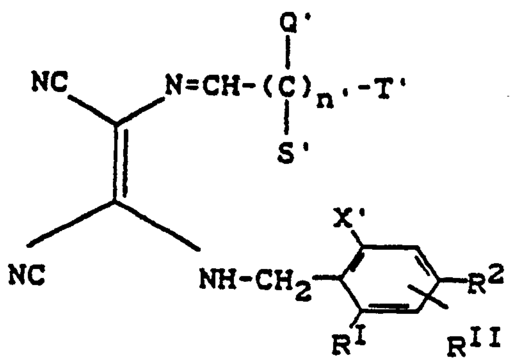

($I_{D'}$)

in der
Q′, S′ und T′ gleich oder verschieden sein können und für Wasserstoff, Fluor, Chlor, Brom, Methyl, Methylsulfonyl, 2-Chlorphenyl, 2,6-Dichlorphenyl,
n′ für 1 oder 2,
X′ und $R^I$ gleich oder verschieden sein können und für Fluor, Chlor, Brom, Iod oder $CF_3$ stehen
und
$R^{II}$ für Wasserstoff, Chlor, Brom, Iod, Cyan, Nitro, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Pentafluorethyl, Trifluorethyl, Trifluormethylsulfon, Trifluormethoxy, Trichlormethoxy, Pentafluorethoxy, Dimethylamino, Diethylamino, Di-β-chlorethylamino, Di-β-hydroxyethylamino und
$R^2$ für Wasserstoff, Alkyl($C_1$-$C_4$), Halogenalkyl($C_1$-$C_4$), Alkoxy($C_1$-$C_4$), Halogenalkoxy($C_1$-$C_4$), Halogen, CN, $NO_2$, Dialkyl($C_1$-$C_4$)amino, Alkoxy($C_1$-$C_4$)carbonyl, Alkyl($C_1$-$C_4$)thio, Alkyl($C_1$-$C_4$)thionyl, Dihalogenalkyl($C_1$-$C_4$)amino, Alkyl($C_1$-$C_4$)sulfonyl, OH, SH, $NH_2$ stehen.

Ganz besonders bevorzugt sind Verbindungen der allgemeinen Formel ($I_D''$)

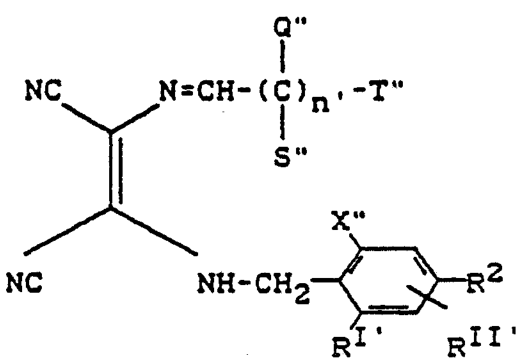

($I_{D''}$)

in der
Q″, S″ und T″ unabhängig voneinander für Wasserstoff, Fluor, Chlor, Brom, Methyl,
n′ für 1 oder 2,
X″ und $R^I$ gleich oder verschieden sein können und für Chlor oder Brom stehen
und
$R^{II}$ für Chlor, Brom, Iod, Wasserstoff, Cyan, Nitro, Methyl, Ethyl, n- und i-Propyl, Methoxy, Ethoxy, Methoxycarbonyl, Ethoxycarbonyl, Trifluormethyl, Trichlormethyl, Dimethylamino, Diethylamino, Trifluormethylsulfon und
$R^2$ für Wasserstoff, Alkyl($C_1$-$C_4$), Halogenalkyl($C_1$-$C_4$), Alkoxy($C_1$-$C_4$), Halogenalkoxy($C_1$-$C_4$), Halogen, CN, $NO_2$, Dialkyl($C_1$-$C_4$)amino, Alkoxy($C_1$-$C_4$)carbonyl, Alkyl($C_1$-$C_4$)thio, Alkyl($C_1$-$C_4$)thionyl, Dihalogenalkyl($C_1$-$C_4$)amino, Alkyl($C_1$-$C_4$) sulfonyl, OH, SH, $NH_2$ stehen.

Von ganz besonderem Interesse sind Verbindungen der allgemeinen Formel ($I_D'''$)

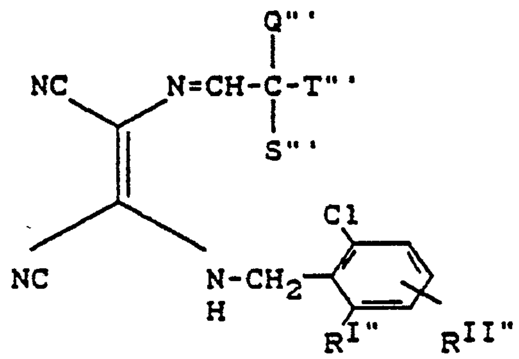

($I_{D'''}$)

in der

Q''', S''' und T''' unabhängig voneinander für Fluor, Chlor, Brom oder Methyl,

$R^{I''}$ für Chlor oder Brom steht und

$R^{II''}$ für Wasserstoff, Chlor, Methyl, Trichlormethyl, Trifluormethyl, Methoxy, Trichlormethoxy oder Trifluormethoxy stehen.

Verwendet man beispielsweise die N-2,6-Dichlorbenzyl-Verbindung des 2,3-Diaminomaleinsäurenitrils und Pivalinaldehyd als Ausgangsprodukte, so kann das Herstellungsverfahren zum Erhalt der Verbindung $I_D$ wie folgt dargestellt werden.

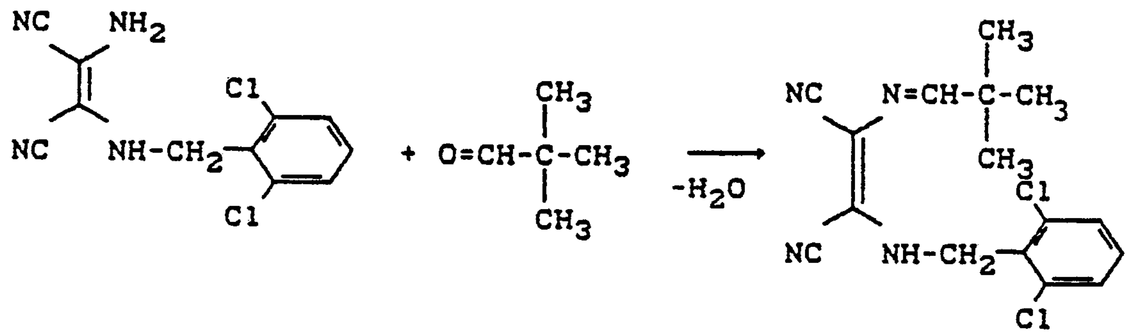


Die zur Durchführung des erfindungsgemäßen Verfahrens als Ausgangsstoffe benötigten Aldehyde sind durch die Formel (III) allgemein definiert. Es handelt sich im wesentlichen um bekannte Verbindungen. Siehe dazu z.B. O. Bayer, in Houben-Weyl, Bd. VII/1, S. 16-36 (1954).

Als Verdünnungsmittel zur Durchführung des erfindungsgemäßen Verfahrens kommen vorzugsweise polare organische Lösungsmittel in Frage, wie. z. B. Alkohole (insbesondere Methanol, Ethanol, Propanol (n und i)), Dimethylformamid (DMF), Dimethylsulfoxid (DMSO), Hexamethylphosphorsäuretriamid (HMPT), Acetonitril.

Die Reaktionstemperatur liegt im allgemeinen im Bereich zwischen ca. 0 °C bis maximal zum Siedepunkt des jeweiligen Lösungsmittels, insbesondere wird die Reaktion bei Temperaturen von ca. 20 bis ca. 100 °C durchgeführt.

Die Umsetzung wird vorzugsweise bei Normaldruck durchgeführt.

Zur Durchführung des erfindungsgemäßen Verfahrens setzt man bevorzugt äquimolare Mengen der Reaktionspartner (X) und (III) miteinander um. Es kann aber auch ein leichter Überschuß eines der beiden Reaktionspartner angewendet werden.

In einer bevorzugten Ausführungsform gibt man bei Raumtemperatur die beiden Reaktionspartner im Verdünnungsmittel zusammen und erhitzt anschließend zum Rückfluß.

Die Aufarbeitung erfolgt nach üblichen Methoden, nach dem Abkühlen wird das Reaktionsprodukt vorzugsweise abgesaugt und nach an sich bekannten Methoden aufgearbeitet.

Die Verbindungen der Formel (X)

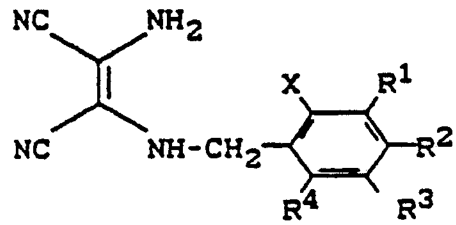

(X)

worin

X, $R^1$, $R^2$, $R^3$ und $R^4$ die oben angegebene Bedeutung besitzen, sind Gegenstand einer älteren im Hinblick auf die vorliegende Anmeldung nicht vorveröffentlichen Patentanmeldung.

Die teilweise neuen Verbindungen der Formel (IX)

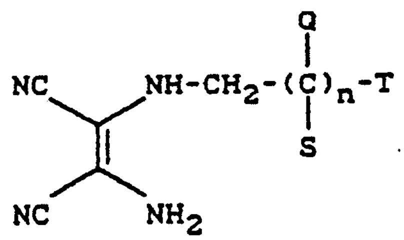

(IX)

in welcher

Q, S, T und n die unter A) angegebene Bedeutung haben, erhält man, wenn man die teilweise neuen Azomethine von 2,3-Diaminomaleinsäurenitril der allgemeinen Formel (IV)

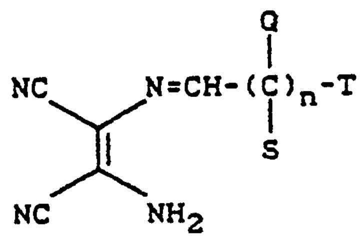

(IV)

in welcher

Q, S, T und n die unter A) angegebene Bedeutung haben, in einem geeigneten Lösungsmittel reduziert.

Die neuen Verbindungen der Formel (XIA)

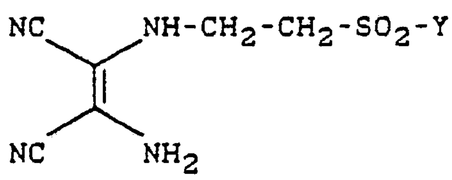

(IXA)

in der

Y für gegebenenfalls substituiertes Alkyl oder Aryl steht, erhält man, wenn man das 2,3-Diaminomaleinsäurenitril der Formel (II)

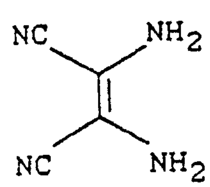

(II)

in einem geeigneten Lösungsmittel mit einem Vinylsulfon bzw. deren Vorläufer der allgemeinen Formel (XI) bzw. (XII)

$CH_2 = CH\text{-}SO_2\text{-}Y$ (XI)

$L\text{-}CH_2\text{-}CH_2\text{-}SO_2\text{-}Y$ (XII)

in der
Y die oben angegebene Bedeutung hat und L eine Abgangsgruppe, wie z.B. $-OSO_3H$, Cl, Br, J bedeutet, umsetzt.

Schließlich wurde gefunden, daß die N-Alkyl-Derivate der allgemeinen Formel (IX) stark ausgeprägte akarizide Eigenschaften besitzen.

Überraschenderweise zeigen die N-Alkyl-Derivate der allgemeinen Formel (IX) eine akarizide Wirksamkeit. Eine derartige Wirkung ist von der Stoffklasse der N-Alkyl-Derivate des Diaminomaleinsäurenitrils bisher noch nicht bekannt geworden. Die N-Alkyl-Verbindungen der allgemeinen Formel (IX) stellen somit eine Bereicherung des Standes der Technik dar.

Die Verbindungen der allgemeinen Formel (IX) können in den stereoisomeren cis- und/oder trans-Formen vorliegen, z.B.

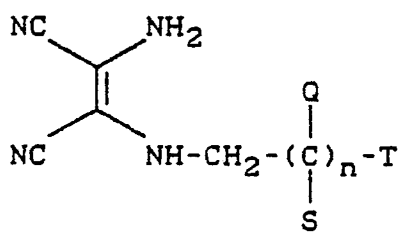

(cis)

bzw.

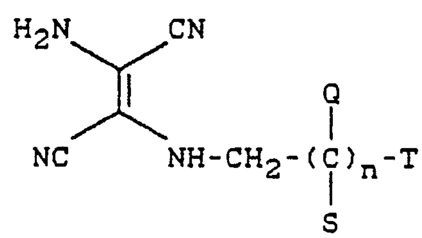

(trans)

Vorzugsweise liegen die Verbindungen jedoch in der cis-Form vor.

In der Formel (IX) stehen Q, S und T, welche gleich oder verschieden sein können, vorzugsweise für Wasserstoff, Fluor, Chlor, Brom, Iod, Methyl, Ethyl, n- und i-Propyl, n-, i-, s-, t-Butyl, $CF_3$, halogensubstituiertes Aryl, gegebenenfalls substituiertes Alkyl- oder Arylsulfonyl. Für n stehen die Zahlen 0, 1, 2, 3, 4, 5 und 6 bevorzugt.

Ganz besonders bevorzugt sind Verbindungen der allgemeinen Formel (IX), in denen unabhängig voneinander Q, S und T für Wasserstoff, Fluor, Chlor, Brom, $CF_3$, Methyl, Ethyl, 2-Chlorphenyl, 2,6-Dichlorphenyl, Methylsulfonyl, halogensubstituiertes Arylsulfonyl und n für die Zahlen 0, 1, 2, 3 und 4 stehen.

Außerordentlich bevorzugt sind Verbindungen der allgemeinen Formel (IX′)

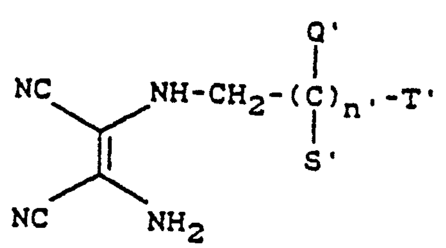

(IX′)

in der
Q′, S′ und T′ unabhängig voneinander für Fluor, Chlor, Brom, Methyl, Methylsulfonyl,
n′ für 1 oder 2 stehen.

Die zur Durchführung des erfindungsgemäßen Verfahrens als Ausgangsstoffe benötigten Azomethine der Formel (IV) können nach den in der Patentschrift JP 51 151 325 dargelegten Verfahren erhalten werden.

Als Verdünnungsmittel zur Durchführung des erfindungsgemäßen Verfahrens kommen sowohl polar

organische Lösungsmittel infrage, wie z. B. Alkohole (insbesondere Methanol, Ethanol, Propanol (n und i), als auch Dimethylformamid (DMF), Dimethylsulfoxid (DMSO), Hexamethylphosphorsäuretriamid (HMPT), Acetonitril, Diethylether, Tetrahydrofuran.

Die Reaktionstemperatur liegt im allgemeinen im Bereich zwischen ca. 0 °C bis maximal zum Siedepunkt des jeweiligen Lösungsmittels, insbesondere wird die Reaktion bei Temperaturen von ca. 10 bis ca. 50 °C durchgeführt.

Die Umsetzung wird vorzugsweise bei Normaldruck durchgeführt.

Als Reduktionsmittel finden Metallhydride Verwendung z.B. des Aluminiums, Zinns und Bors.

Bevorzugt ist der Einsatz von Natriumborhydrid in protischen Lösungsmitteln wie z.B. Methanol, Ethanol oder in aprotischen Lösungsmitteln wie z.B. DMF, THF oder Hexamethylphosphorsäuretriamid (HMPT).

Die Aufarbeitung erfolgt nach üblichen Methoden, nach dem Austragen auf Eis bzw. Wasser wird das Produkt abgesaugt bzw. extrahiert.

Das Herstellungsverfahren zum Erhalt der Verbindung der Formel (IX) kann wie folgt an einem Beispiel dargestellt werden:

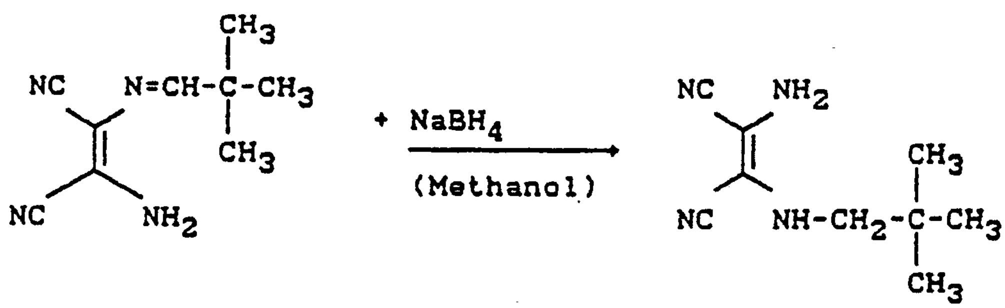


Im einzelnen seien außer den bei den Herstellungsbeispielen genannten Verbindungen die folgenden Bisazomethine des 2,3-Diaminomaleinsäurenitrils der Formel ($I_D$) beispielhaft genannt:

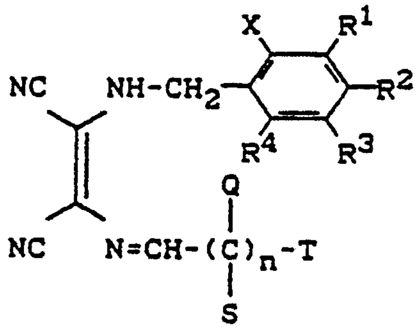

$(I_D)$

| Q | S | T | n | X | $R^1$ | $R^2$ | $R^3$ | $R^4$ |
|---|---|---|---|---|---|---|---|---|
| - | - | H | 0 | Cl | H | H | H | Cl |
| - | - | H | 0 | F | H | H | H | Cl |
| - | - | H | 0 | F | H | H | H | F |
| - | - | H | 0 | Br | H | H | H | Br |
| - | - | H | 0 | Cl | H | Cl | H | Cl |
| $CH_3$ | $CH_3$ | $CH_3$ | 1 | Cl | H | H | H | Cl |
| $CH_3$ | $CH_3$ | $CH_3$ | 1 | Br | H | H | H | Br |
| $C_2H_5$ | H | $C_2H_5$ | 1 | Cl | H | H | H | Cl |
| $C_2H_5$ | $CH_3$ | $C_2H_5$ | 1 | Cl | H | H | H | Cl |
| $CH_3$ | $CH_3$ | $CH_3$ | 1 | H | $CF_3$ | H | H | H |
| $CH_3$ | $CH_3$ | $CH_3$ | 1 | H | Cl | Cl | H | H |
| H | H | H | 1 | Cl | H | H | H | Cl |

| Q | S | T | n | X | $R^1$ | $R^2$ | $R^3$ | $R^4$ |
|---|---|---|---|---|---|---|---|---|
| H | H | H | 1 | Cl | H | Cl | H | H |
| H | H | H | 1 | Br | H | H | H | Br |
| Cl | Cl | Cl | 1 | Cl | H | H | H | Cl |
| Br | Br | Br | 1 | Cl | H | H | H | Cl |
| Cl | Cl | $OCH_3$ | 1 | Cl | H | H | H | Cl |
| Cl | Cl | $OCH_3$ | 1 | Cl | H | H | H | Cl |
| Br | Br | $OCH_3$ | 1 | Cl | H | H | H | Cl |
| F | F | $OCH_3$ | 1 | Cl | H | H | H | Cl |
| F | F | $OC_2H_5$ | 1 | Cl | H | H | H | Cl |
| F | F | $OC_2H_5$ | 1 | F | H | H | H | Cl |
| F | F | $OC_2F_4$-$OCF_3$ | 1 | Cl | H | H | H | Cl |
| F | F | $OCF_3$ | 1 | Cl | H | H | H | Cl |
| Cl | Cl | $OCH_3$ | 1 | F | H | H | H | F |
| Cl | Cl | $OCH_3$ | 1 | F | H | H | H | Cl |
| F | F | $OCF_3$ | 1 | $CF_3$ | H | H | H | $CF_3$ |
| Br | Br | $OC_2H_4OCH_3$ | 1 | Cl | H | H | H | Cl |
| Br | Br | $OCH_3$ | 1 | Cl | H | H | H | F |
| Br | Br | $OCH_3$ | 1 | F | $OCF_3$ | F | F | F |
| Cl | Cl | Cl | 1 | F | $OCF_3$ | F | F | F |
| Br | Br | Br | 1 | F | $OCF_3$ | F | F | F |
| - | - | H | 0 | F | $OCF_3$ | F | F | F |
| $CH_3$ | $CH_3$ | $CH_3$ | 1 | F | $OCF_3$ | F | F | F |
| $C_2H_5$ | H | $C_2H_5$ | 1 | F | $OCF_3$ | F | F | F |
| $C_2H_5$ | $CH_3$ | $C_2H_5$ | 1 | F | $OCF_3$ | F | F | F |
| H | H | H | 1 | F | $OCF_3$ | F | F | F |
| $CH_3$ | $CH_3$ | $CH_3$ | 1 | Cl | H | F | H | Cl |
| $CH_3$ | H | $CH_3$ | 1 | Cl | H | H | H | Cl |
| $CH_3$ | H | $CH_3$ | 1 | Br | H | H | H | Br |

| Q | S | T | n | X | $R^1$ | $R^2$ | $R^3$ | $R^4$ |
|---|---|---|---|---|---|---|---|---|
| F | F | F | 1 | Cl | H | H | H | Cl |
| Cl | Cl | Cl | 1 | $CF_3$ | H | H | H | $CF_3$ |
| Cl | Cl | Cl | 1 | Br | H | H | H | Br |
| Br | Br | Br | 1 | Cl | H | H | H | F |
| Br | Br | Br | 1 | F | H | H | H | F |
| $CH_3$ | $CH_3$ | $CH_3$ | 1 | $CF_3$ | H | H | H | $CF_3$ |
| Cl | Cl | Cl | 1 | Cl | H | Cl | H | Cl |
| Br | Br | Br | 1 | Cl | H | Cl | H | Cl |
| F | F | F | 1 | Cl | H | Cl | H | Cl |
| Cl | Cl | Cl | 1 | F | $OCF_3$ | F | F | F |
| 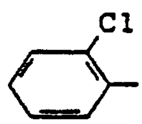 | H | H | 1 | Cl | H | H | H | Cl |
| 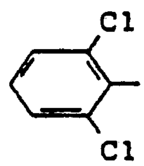 | H | H | 1 | Cl | H | H | H | Cl |
| 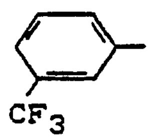 | H | H | 1 | Cl | H | H | H | Cl |
| 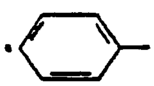 | H | H | 1 | Cl | H | H | H | Cl |
| 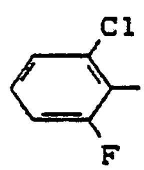 | H | H | 1 | Cl | H | H | H | Cl |

| Q | S | T | n | X | $R^1$ | $R^2$ | $R^3$ | $R^4$ |
|---|---|---|---|---|---|---|---|---|
| 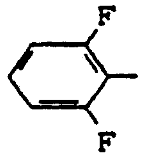 | H | H | 1 | Cl | H | H | H | Cl |
| 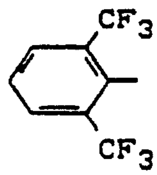 | H | H | 1 | Cl | H | H | H | Cl |
| 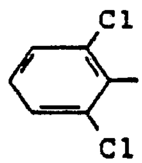 | H | H | 1 | Cl | H | H | H | H |
| 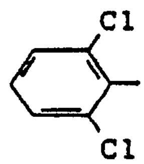 | H | H | 1 | H | Cl | Cl | H | H |

Man erhält die neuen, N,N′-disubstituierten Derivate des 2,3-Diaminomaleinsäurenitrils der Formel ($I_E$)

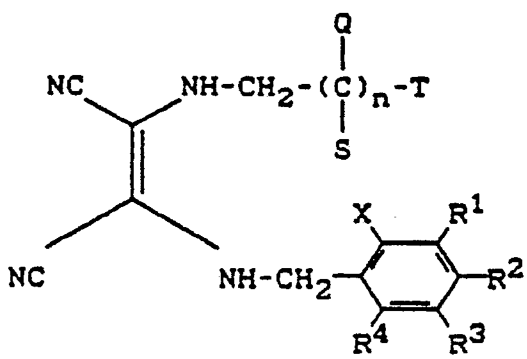

($I_E$)

in welcher
Q, S, T, n, X, $R^1$, $R^2$, $R^3$ und $R^4$ die unter B) angegebene Bedeutung haben, wenn man die Bisazomethine der allgemeinen Formel ($I_B$)

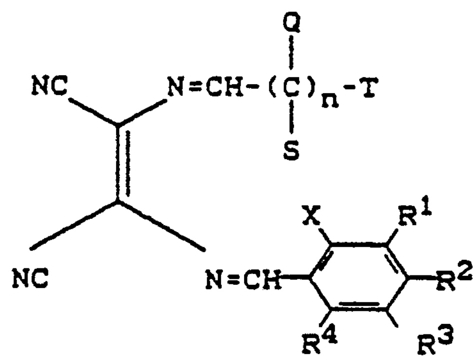

$(I_B)$

oder die Amino-imino-Derivate des 2,3-Diaminomaleinsäurenitrils der Formel ($I_C$)

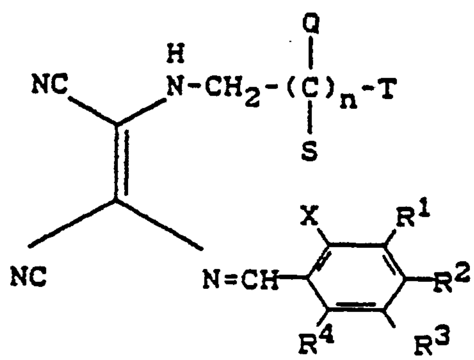

$(I_C)$

bzw. der Formel ($I_D$)

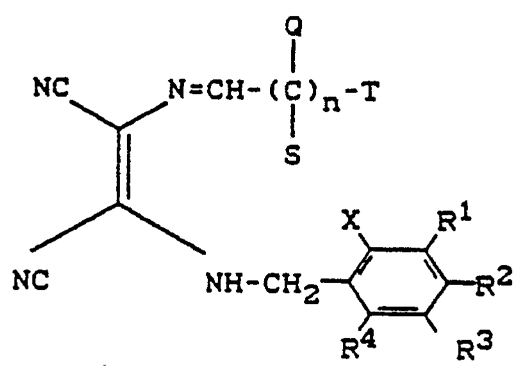

$(I_D)$

in welcher

Q, S, T, n, X, $R^1$, $R^2$, $R^3$ und $R^4$ die unter B) angegebene Bedeutung haben, in einem geeigneten Verdünnungsmittel reduziert.

Die Verbindungen der allgemeinen Formel ($I_E$) können in den stereoisomeren cis- und/oder trans-Formen vorliegen. Vorzugsweise liegen die Verbindungen jedoch in der cis-Form vor.

Die überraschenderweise akarizid wirksamen N,N′-disubstituierten Derivate des 2,3-Diaminomaleinsäurenitrils sind durch die Formel ($I_E$) allgemein definiert.

Die Reste Q, S und T stehen vorzugsweise unabhängig voneinander für Wasserstoff, Fluor, Chlor, Brom, Iod, Methyl, Ethyl, n- und i-Propyl, n-, i-, s-, t-Butyl, $CF_3$, halogensubstituiertes Aryl, gegebenenfalls substituiertes Alkyl- und Arylsulfonyl. Für n sind die Zahlen 0, 1, 2, 3, 4, 5 und 6 bevorzugt.

X steht vorzugsweise für Wasserstoff, Fluor, Chlor, Brom, Iod, $CF_3$ oder CN.

Bei den Resten $R^1$, $R^2$, $R^3$ und $R^4$, welche ebenfalls gleich oder verschieden sein können, kommen als Substituenten für Alkyl, Alkoxy, Dialkylamino, Alkylthio, Alkylthionyl, Alkylsulfonyl vorzugsweise infrage: Halogen, insbesondere Fluor, Chlor und Brom sowie OH, $NH_2$.

Bevorzugt sind Verbindungen der Formel ($I_E$), in welcher unabhängig voneinander n für die Zahl 0, 1, 2, 3 oder 4 steht, Q, S oder T unabhängig voneinander für Wasserstoff, Fluor, Chlor, Brom, $CF_3$, Methyl,

Ethyl, Phenyl, 2-Chlorphenyl, 2,6-Dichlorphenyl, Methylsulfonyl, Trifluormethylsulfonyl, halogensubstituiertes Arylsulfonyl,
X und $R^4$ gleich oder verschieden sein können und für Fluor, Chlor, Brom, Iod, $CF_3$ oder CN stehen,
$R^1$, $R^2$ und $R^3$ gleich oder verschieden sein können und für Wasserstoff, Alkyl($C_1$-$C_4$), Halogenalkyl($C_1$-$C_4$), Alkoxy($C_1$-$C_4$), Halogenalkoxy($C_1$-$C_4$), Halogen, CN, $NO_2$, Dialkyl($C_1$-$C_4$)amino, Alkoxy($C_1$-$C_4$)carbonyl, Alkyl($C_1$-$C_4$)thio, Alkyl($C_1$-$C_4$)thionyl, Dihalogenalkyl($C_1$-$C_4$)amino, Alkyl($C_1$-$C_4$)sulfonyl, OH, SH, $NH_2$ stehen.

Ganz besonders bevorzugt sind Verbindungen der allgemeinen Formel ($I_E'$)

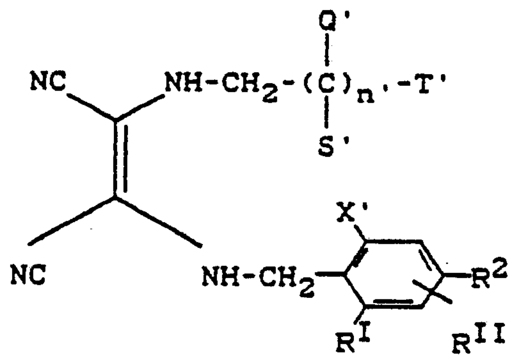

($I_{E'}$)

in der
Q′, S′ und T′ gleich oder verschieden sein können und für Wasserstoff, Fluor, Chlor, Brom, Methyl, Methylsulfonyl, 2-Chlorphenyl, 2,6-Dichlorphenyl,
n′ für 1 oder 2,
X′ und $R^I$ gleich oder verschieden sein können und für Fluor, Chlor, Brom, Iod oder $CF_3$ stehen
und
$R^{II}$ für Wasserstoff, Chlor, Brom, Iod, Cyan, Nitro, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Pentafluorethyl, Trifluorethyl, Trifluormethylsulfon, Trifluormethoxy, Trichlormethoxy, Pentafluorethoxy, Dimethylamino, Diethylamino, Di-β-chlorethylamino, Di-β-hydroxyethylamino und
$R^2$ für Wasserstoff, Alkyl($C_1$-$C_4$), Halogenalkyl($C_1$-$C_4$), Alkoxy($C_1$-$C_4$), Halogenalkoxy($C_1$-$C_4$), Halogen, CN, $NO_2$, Dialkyl($C_1$-$C_4$)amino, Alkoxy($C_1$-$C_4$)carbonyl, Alkyl($C_1$-$C_4$)thio, Alkyl($C_1$-$C_4$)thionyl, Dihalogenalkyl($C_1$-$C_4$)amino, Alkyl($C_1$-$C_4$)sulfonyl, OH, SH, $NH_2$ stehen.

Außerordentlich bevorzugt sind Verbindungen der allgemeinen Formel ($I_E''$)

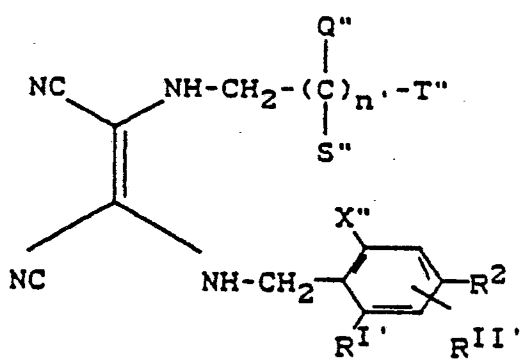

($I_{E''}$)

in der
Q″, S″ und T″ unabhängig voneinander für Wasserstoff, Fluor, Chlor, Brom, Methyl,
n′ für 1 oder 2,
X″ und $R^I$ gleich oder verschieden sein können und für Chlor oder Brom stehen
und
$R^{II}$ für Chlor, Brom, Iod, Wasserstoff, Cyan, Nitro, Methyl, Ethyl, n- und i-Propyl, Methoxy, Ethoxy, Methoxycarbonyl, Ethoxycarbonyl, Trifluormethyl, Trichlormethyl, Dimethylamino, Diethylamino, Trifluormethylsulfon und

$R^2$ für Wasserstoff, Alkyl($C_1$-$C_4$), Halogenalkyl($C_1$-$C_4$), Alkoxy($C_1$-$C_4$), Halogenalkoxy($C_1$-$C_4$), Halogen, CN, $NO_2$, Dialkyl($C_1$-$C_4$)amino, Alkoxy($C_1$-$C_4$)carbonyl, Alkyl($C_1$-$C_4$)thio, Alkyl($C_1$-$C_4$)thionyl, Dihalogenalkyl($C_1$-$C_4$)amino, Alkyl($C_1$-$C_4$)sulfonyl, OH, SH, $NH_2$ stehen.

Von ganz besonderem Interesse sind Verbindungen der allgemeinen Formel ($I_E'''$)

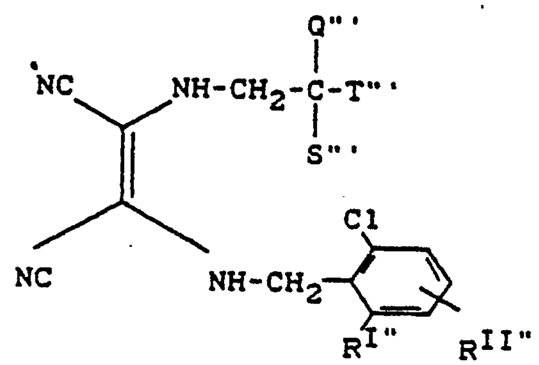

($I_E'''$)

in der

Q‴, S‴ und T‴ unabhängig voneinander für Fluor, Chlor, Brom oder Methyl,

$R^{I}$″ für Chlor oder Brom steht und

$R^{II}$ für Wasserstoff, Chlor, Methyl, Trichlormethyl, Trifluormethyl, Methoxy, Trichlormethoxy oder Trifluormethoxy stehen.

Im einzelnen seien außer den bei den Herstellungsbeispielen genannten Verbindungen die folgenden Bisazomethine des 2,3-Diaminomaleinsäurenitrils der Formel ($I_E$) beispielhaft genannt:

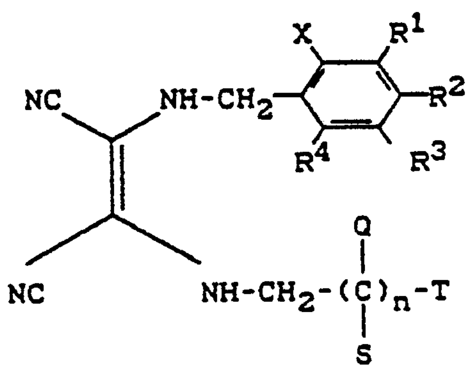

($I_E$)

| Q | S | T | n | X | $R^1$ | $R^2$ | $R^3$ | $R^4$ |
|---|---|---|---|---|---|---|---|---|
| H | H | $-SO_2CH_3$ | 1 | Cl | H | H | H | Cl |
| H | H | $-SO_2CH_3$ | 1 | Cl | H | H | H | F |
| H | H | $-SO_2CH_3$ | 1 | F | H | H | H | F |
| H | H | $-SO_2CH_3$ | 1 | Br | H | H | H | Br |
| H | H | $-SO_2CF_3$ | 1 | Cl | H | H | H | Cl |

| Q | S | T | n | X | $R^1$ | $R^2$ | $R^3$ | $R^4$ |
|---|---|---|---|---|---|---|---|---|
| H | H | $-SO_2CF_3$ | 1 | $CF_3$ | H | H | H | $CF_3$ |
| H | - | - | 0 | Cl | H | H | H | Cl |
| - | - | H | 0 | Cl | H | H | H | F |
| - | - | H | 0 | Br | H | H | H | Br |
| - | - | H | 0 | Cl | H | H | H | Br |
| - | - | H | 0 | Cl | H | F | H | Cl |
| H | H | H | 1 | Cl | H | H | H | Cl |
| H | H | H | 1 | Cl | H | Cl | H | Cl |
| H | H | H | 1 | $CF_3$ | H | H | H | $CF_3$ |
| $CH_3$ | $CH_3$ | $CH_3$ | 1 | Cl | H | H | H | Cl |
| $C_2H_5$ | H | $C_2H_5$ | 1 | Cl | H | H | H | Cl |
| $C_2H_5$ | $CH_3$ | $C_2H_5$ | 1 | Cl | H | H | H | Cl |
| H | H | $CH_3$ | 1 | Cl | H | H | H | Cl |
| $CH_3$ | H | $CH_3$ | 1 | Cl | H | H | H | Cl |
| Cl | Cl | Cl | 1 | Cl | H | H | H | Cl |
| Br | Br | Br | 1 | Cl | H | H | H | Cl |
| Br | Br | Br | 1 | Cl | H | H | H | F |
| Cl | Cl | Cl | 1 | Cl | H | H | H | F |
| Cl | Cl | Cl | 1 | Br | H | H | H | Br |
| Cl | Cl | Cl | 1 | H | $CF_3$ | H | H | H |
| Cl | Cl | Cl | 1 | $CF_3$ | H | H | H | $CF_3$ |
| Br | Br | Br | 1 | $CF_3$ | H | H | H | $CF_3$ |
| F | F | $OCF_3$ | 1 | Cl | H | H | H | Cl |
| F | F | $OCF_3$ | 1 | Br | H | H | H | Br |
| F | F | $OCF_3$ | 1 | Cl | H | H | H | F |
| F | F | $OCF_3$ | 1 | Fl | H | H | H | F |
| H | H | $OCH_3$ | 1 | Cl | H | H | H | Cl |
| H | H | $OCH_3$ | 1 | Cl | H | H | H | F |
| H | H | $OCH_3$ | 1 | Br | H | H | H | Br |

| Q | S | T | n | X | $R^1$ | $R^2$ | $R^3$ | $R^4$ |
|---|---|---|---|---|---|---|---|---|
| 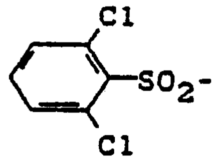 | H | H | 1 | Cl | H | H | H | Cl |
| 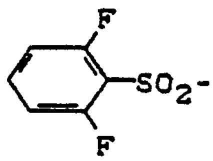 | H | H | 1 | Cl | H | H | H | Cl |
| 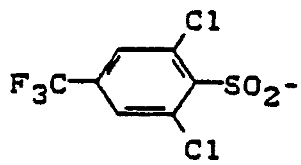 | H | H | 1 | Cl | H | H | H | Cl |
| 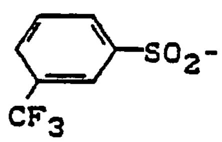 | H | H | 1 | Cl | H | H | H | Cl |
| 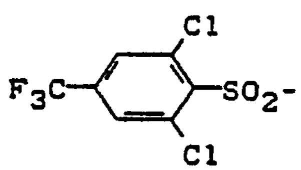 | H | H | 1 | Br | H | H | H | Br |
| 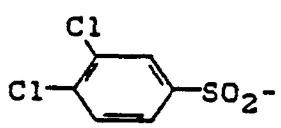 | H | H | 1 | Cl | H | H | H | Cl |
| 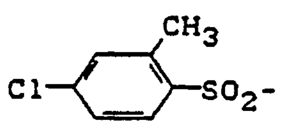 | H | H | 1 | Cl | H | H | H | Cl |
| 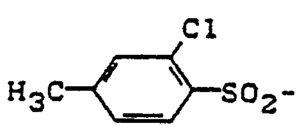 | H | H | 1 | Cl | H | H | H | Cl |

| Q | S | T | n | X | $R^1$ | $R^2$ | $R^3$ | $R^4$ |
|---|---|---|---|---|---|---|---|---|
| 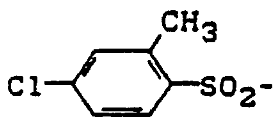 | H | H | 1 | F | H | H | H | Cl |
| 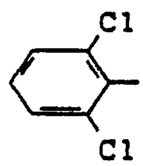 | H | H | 1 | Cl | H | H | H | Cl |
| 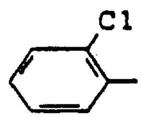 | H | H | 1 | Cl | H | H | H | Cl |
| 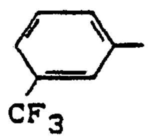 | H | H | 1 | Cl | H | H | H | Cl |
| 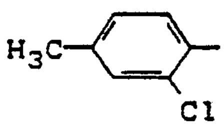 | H | H | 1 | Cl | H | H | H | Cl |
| 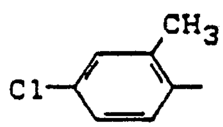 | H | H | 1 | Br | H | H | H | Br |

Man erhält die neuen N,N′-disubstituierten Derivate des 2,3-Diaminomaleinsäurenitrils der Formel ($I_F$)

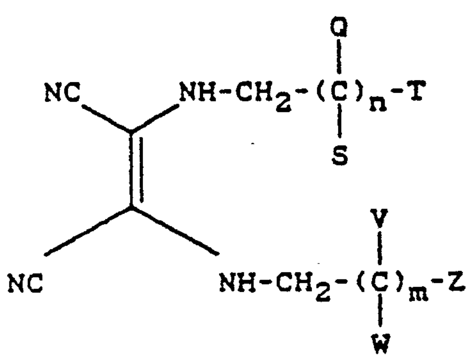

($I_F$)

in welcher
Q, S, T, V, W, Z, n und m die unter A) angegebene Bedeutung haben, wenn man Bisazomethine der allgemeinen Formel ($I_A$)

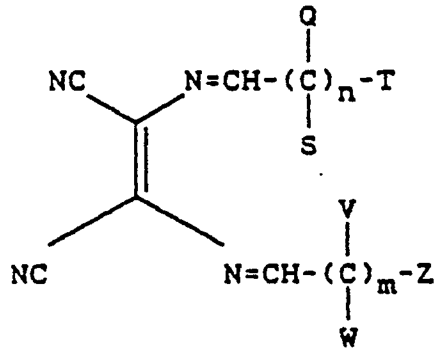

$(I_A)$

oder die Amino-imino-Derivate des 2,3-Diaminomaleinsäurenitrils der Formel (XIII)

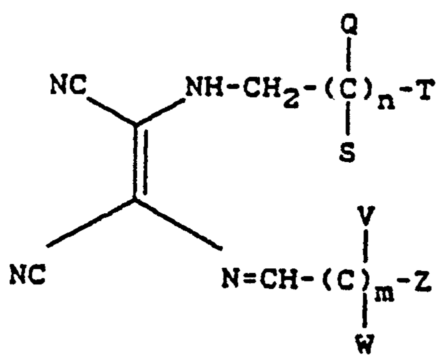

(XIII)

bzw. der Formel (XIV)

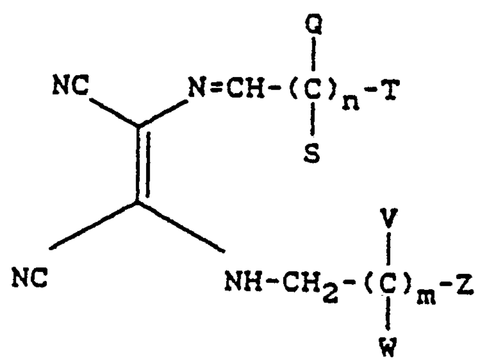

(XIV)

in welcher

Q, S, T, V, W, Z, n und m die unter A) angegebene Bedeutung haben, in einem geeigneten Verdünnungsmittel reduziert.

Die Verbindungen der allgemeinen Formel $(I_F)$ können in den stereoisomeren cis- und/oder trans-Formen vorliegen. Vorzugsweise liegen die Verbindungen jedoch in der cis-Form vor.

Die überraschenderweise akarizid wirksamen N,N′-disubstituierten Derivate des 2,3-Diaminomaleinsäurenitrils sind durch die Formel $(I_F)$ allgemein definiert.

Die Reste Q, S, T, V, W, Z stehen vorzugsweise unabhängig voneinander für Wasserstoff, Fluor, Chlor, Brom, Iod, Methyl, Ethyl, n- und i-Propyl, n-, i-, s-, t-Butyl, $CF_3$, halogensubstituiertes Aryl, gegebenenfalls substituiertes Alkyl- und Arylsulfonyl. Für n und m sind die Zahlen 1, 2, 3, 4, 5 und 6 bevorzugt.

Ganz besonders bevorzugt sind Verbindungen der Formel $(I_F)$, in welcher unabhängig voneinander n und m für die Zahl 0, 1, 2, 3 oder 4, Q, S, T, V, W oder Z unabhängig voneinander für Wasserstoff, Fluor, Chlor, Brom, $CF_3$, Methyl, Ethyl, Phenyl, 2-Chlorphenyl, 2,6-Dichlorphenyl, Methylsulfonyl, Trifluormethylsulfonyl, halogensubstituiertes Arylsulfonyl steht.

Von ganz starkem Interesse sind Verbindungen der allgemeinen Formel $(I_F')$

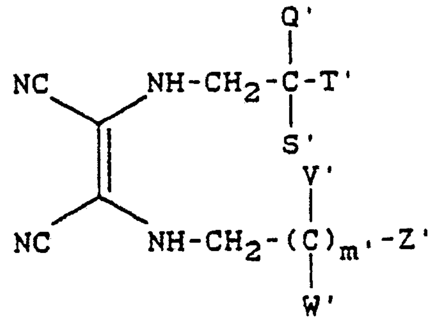

$(I_{F'})$

in der unabhängig voneinander Q′, S′, T′, V′, W′ und Z′ für Wasserstoff, Fluor, Chlor, Brom, $CF_3$, Methyl, 2-Chlorphenyl, 2,6-Dichlorphenyl, Methylsulfonyl, Trifluormethylsulfonyl, halogensubstituiertes Arylsulfonyl und m′ für die Zahl 0, 1, 2 oder 3 stehen.

Außerordentlich stark bevorzugt sind Verbindungen der allgemeinen Formel ($I_{F''}$)

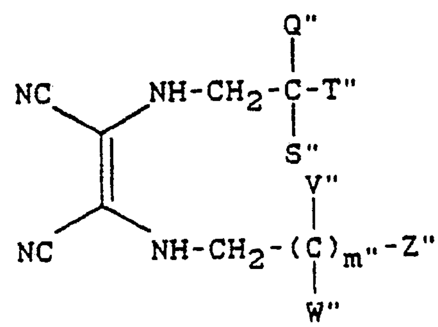

$(I_{F''})$

in der unabhängig voneinander Q″, S″, T″, V″, W″ und Z″ für Wasserstoff, Fluor, Chlor, Brom, $CF_3$, Methyl, Methylsulfonyl, Trifluormethylsulfonyl, 2-Chlorphenylsulfonyl, 2,6-Dichlorphenylsulfonyl, und m″ für die Zahl 0, 1 oder 2 steht.

Als Verdünnungsmittel zur Durchführung des erfindungsgemäßen Verfahrens zur Herstellung von Verbindungen der Formel ($I_F$) kommen sowohl polare organische Lösungsmittel infrage, wie z.B. Alkohole (insbesondere Methanol, Ethanol, Propanol (n und i), als auch Dimethylformamid (DMF), Dimethylsulfoxid (DMSO), Hexamethylphosphorsäuretriamid (HMPT), Acetonitril, Diethylether, Tetrahydrofuran.

Die Reaktionstemperatur liegt im allgemeinen im Bereich zwischen ca. 0° C bis maximal zum Siedepunkt des jeweiligen Lösungsmittels, insbesondere wird die Reaktion bei Temperaturen von ca. 10 bis ca. 50° C durchgeführt.

Die Umsetzung wird vorzugsweise bei Normaldruck durchgeführt.

Als Reduktionsmittel finden Metallhydride Verwendung z.B. des Aluminiums, Zinns und Bors.

Bevorzugt wird Natriumborhydrid in protischen Lösungsmitteln wie z.B. Methanol, Ethanol oder in aprotischen Lösungsmitteln wie z.B. DMF, Tetrahydrofuran oder Hexamethylphosphorsäuretriamid (HMPT).

Die Aufarbeitung erfolgt nach üblichen Methoden, im allgemeinen wird nach dem Austragen auf Eis bzw. Wasser das Produkt abgesaugt bzw. extrahiert.

Das Herstellungsverfahren zum Erhalt der Verbindung der Formel ($I_F$) kann wie folgt an einem Beispiel dargestellt werden:

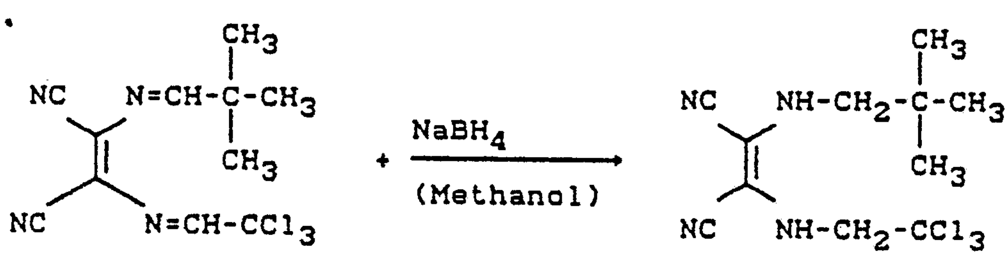

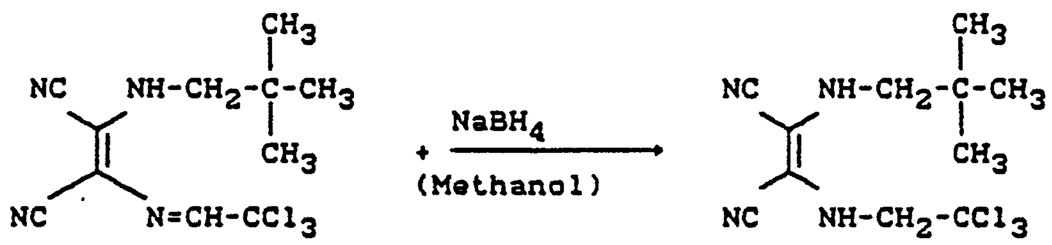


Im einzelnen seien außer den bei den Herstellungsbeispielen genannten Verbindungen die folgenden Bisazomethine des 2,3-Diaminomaleinsäurenitrils der Formel ($I_F$) beispielhaft genannt:

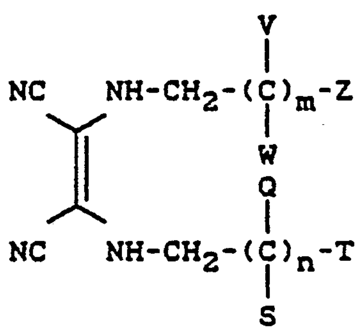

($I_F$)

| Q | S | T | n | V | W | Z | m |
|---|---|---|---|---|---|---|---|
| H | H | H | 1 | $CH_3$ | $CH_3$ | $CH_3$ | 1 |
| $C_2H_5$ | $C_2H_5$ | $CH_3$ | 1 | $CH_3$ | $CH_3$ | $CH_3$ | 1 |
| $C_2H_5$ | $C_2H_5$ | $CH_3$ | 1 | Cl | Cl | Cl | 1 |
| - | - | H | 0 | F | F | F | 1 |
| - | - | H | 0 | Cl | Cl | Cl | 1 |
| - | - | H | 0 | F | F | $OCF_3$ | 1 |
| - | - | H | 0 | $CF_3$ | $CF_3$ | $CF_3$ | 1 |

| Q | S | T | n | V | W | Z | m |
|---|---|---|---|---|---|---|---|
| $CH_3$ | $CH_3$ | H | 1 | F | F | $OCH_3$ | 1 |
| H | H | $CH_3$ | 3 | Br | Br | Br | 1 |
| - | - | H | 0 | F | F | $CF_3$ | 1 |
| $CH_3$ | $CH_3$ | $CH_3$ | 1 | Br | Br | Br | 1 |
| $CH_3$ | $CH_3$ | $CH_3$ | 1 | H | H | $-SO_2CH_3$ | 1 |
| $CH_3$ | $CH_3$ | $CH_3$ | 1 | H | H | $-SO_2CF_3$ | 1 |
| Cl | Cl | Cl | 1 | H | H | $-SO_2CH_3$ | 1 |
| Br | Br | Br | 1 | H | H | $-SO_2CF_3$ | 1 |
| $CH_3$ | $CH_3$ | $CH_3$ | 1 | H | H | $-SO_2$ 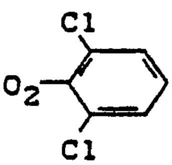 | 1 |
| $CH_3$ | $CH_3$ | $CH_3$ | 1 | H | H | $-SO_2$ 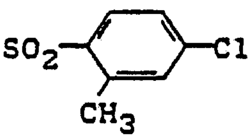 | 1 |
| - | - | H | 0 | H | H | $-SO_2$ 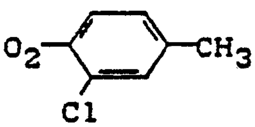 | 1 |
| - | - | H | 0 | H | H | $-SO_2$ 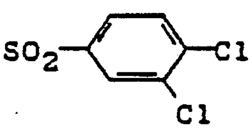 | 1 |
| 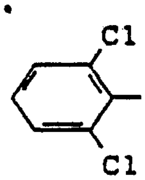 | H | H | 1 | H | H | 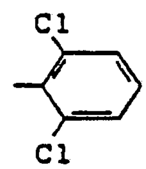 | 1 |
| 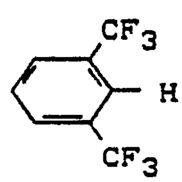 | H | H | 1 | H | H | 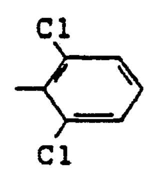 | 1 |

| Q | S | T | n | V | W | Z | m |
|---|---|---|---|---|---|---|---|
| 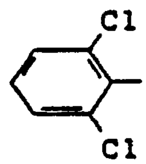 | H | H | 1 | H | H | 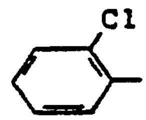 | 1 |
| 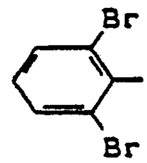 | H | H | 1 | H | H | 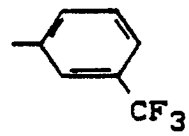 | 1 |
| 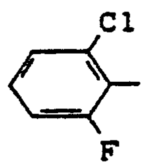 | H | H | 1 | H | H | 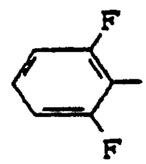 | 1 |
| 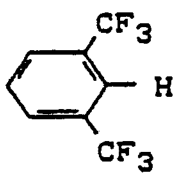 | H | H | 1 | H | H | 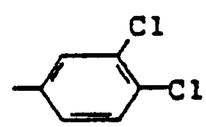 | 1 |
| Cl | Cl | Cl | 1 | H | H | 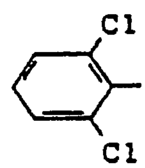 | 1 |
| Br | Br | Br | 1 | H | H | 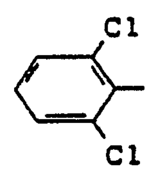 | 1 |
| F | F | F | 1 | H | H | 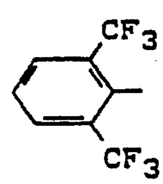 | 1 |

Die Wirkstoffe eignen sich bei guter Pflanzenverträglichkeit und günstiger Warmblütertoxizität zur Bekämpfung von tierischen Schädlingen, insbesondere Milben (Acarina), die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Acarina z. B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben.

Sie sind gegen normalsensible und resistente Arten und Stämme, sowie gegen alle parasitierenden und nicht parasitierenden Entwicklungsstadien der Ektoparasiten wirksam.

Die erfindungsgemäßen Wirkstoffe zeichnen sich durch eine hohe akarizide Wirksamkeit aus. Sie lassen sich mit besonders gutem Erfolg gegen pflanzenschädigende Milben, wie beispielsweise gegen die gemeine Spinnmilbe (Tetranychus urticae) einsetzen.

Darüber hinaus weisen die erfindungsgemäßen Verbindungen noch eine fungizide Wirksamkeit auf.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden oder Fungiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Die erfindungsgemäß verwendbaren Wirkstoffe eignen sich auch zur Bekämpfung von Milben, Zecken

usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäß verwendbaren Wirkstoffe geschieht auf diesem Gebiet in bekannter Weise wie durch orale Anwendung in Form von beispielsweise Tabletten, Kapseln, Tränken, Granulaten, durch dermale bzw. äußerliche Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke) möglich.

Beispiel A

Tetranychus-Test (resistent)

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigten z. B. die folgende Verbindung der Herstellungsbeispiele eine starke Wirksamkeit gegen Spinnmilben: (6).

Herstellungsbeispiele

Beispiel 1

13,25 g des N-2,6-Dichlorbenzyliden-amino-maleinsäurenitrils werden in 100 ml absolutem Ethanol mit 4,3 g Pivalaldehyd und 100 mg p-Toluolsulfonsäure zum Rückfluß erhitzt. Man läßt noch 4 Stunden kochen und trägt dann auf 500 g Eis aus. Durch Absaugen isoliert man 14,6 g der folgenden Verbindung mit dem Schmelzpunkt >150 °C (Zers.):

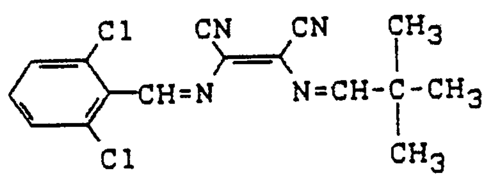

Beispiel 2

Verwendet man im obigen Beispiel Chloral anstelle des Pivalaldehyds und wendet sonst die gleichen Reaktionsbedingungen an, so erhält man folgende Verbindung als gelbes zähflüssiges Öl.

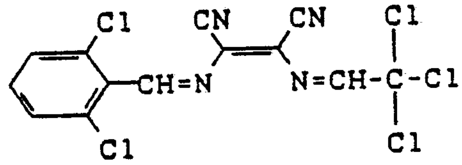

Beispiel 1A

Die in den Beispielen 1 und 2 eingesetzte Ausgangsverbindung N-2,6-Dichlorbenzyliden-amino-maleinsäurenitril der Formel

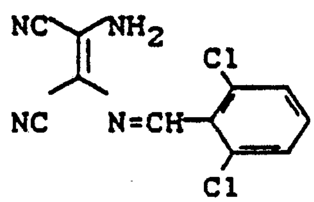

kann wie folgt hergestellt werden:
In 100 ml Acetonitril bringt man eine Mischung aus 10,8 g 2,3-Diaminomaleinsäurenitril und 17,5 g 2,6-Dichlorbenzaldehyd zum Sieden. Die Dünnschichtchromatographie zeigt nach 4 Stunden das Reaktionsende an. Nach dem Abkühlen saugt man 24,2 g (91 % der Theorie) N-2,6-Dichlorbenzylidenaminomaleinsäurenitril ab. Die Substanz (gelbgrüne Nadeln) schmilzt bei 191 °C.

Beispiel 3

Zu 13,35 g N-2,6-Dichlorbenzyl-amino-maleinsäurenitril in 100 ml Acetonitril gibt man 4,3 g Pivalaldehyd und erhitzt zum Rückfluß. Nach 1 Stunde ist die Umsetzung vollständig (DC-Kontrolle). Man kühlt stark ab und saugt die gelben Kristalle ab. Man erhält 14,2 g der folgenden Verbindung, die sich oberhalb von 230 °C allmählich zersetzt.

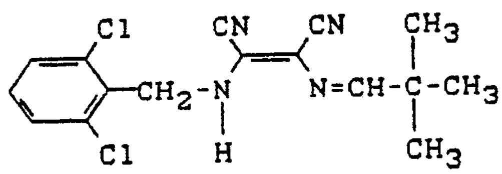

Beispiel 3A

In 200 ml Methanol bringt man eine Mischung aus 10,8 g 2,3-Diaminomaleinsäurenitril und 17,5 g 2,6-Dichlorbenzaldehyd zum Sieden. Die Dünnschichtchromatographie zeigt nach 4 Stunden die Bildung des Azomethins an. Anschließend kühlt man den Reaktionsansatz auf 0 °C ab und gibt portionsweise 4,0 g Natriumborhydrid hinzu. Man läßt 30 Minuten nachrühren, bis eine klare braune Lösung erhalten wird. Nach dünnschichtchromatographischer Kontrolle trägt man den Ansatz auf 500 g Eis aus und saugt das ausgefallene Produkt ab. Man erhält 22,8 g der folgenden Verbindung

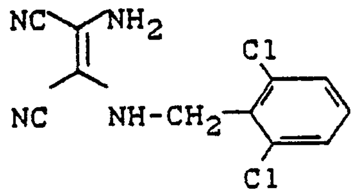

in Form eines gelbbraunen Pulvers vom Schmelzpunkt 159° C (aus Toluol).

Beispiel 4

Man löst 26,6 g N-3-Trifluormethylbenzyl-amino-maleinsäurenitril und 28,1 g Bromal in einer Mischung aus 200 ml Ethanol abs. und 50 ml Toluol. Man erhitzt zum Rückfluß und zieht am Kolonnenkopf langsam das Wasser-Toluol-Ethanol-Gemisch ab. Durch DC-Kontrolle wird das Reaktionsende bestimmt. Nach dem Abkühlen isoliert man das Reaktionsprodukt durch Austragen auf Eiswasser. Man erhält 49,1 g einer amorphen, gelben Masse der folgenden Strukturformel (IR: -NH = 3.300 $cm^{-1}$).

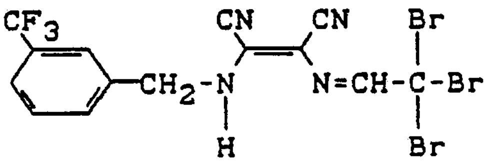

Beispiel 4A

In eine Lösung von 11,88 g 3-Trifluormethylbenzylidendiaminomaleinsäurenitril in 100 ml Methanol gibt man bei 0 bis 5° C 1,8 g Natriumborhydrid. Die Dünnschichtchromatographie zeigt das Reaktionsende an. Der Reaktionsansatz wird auf 500 g Eis ausgetragen und das Produkt abgesaugt. Man erhält 9,2 g eines Stoffs der folgenden Formel

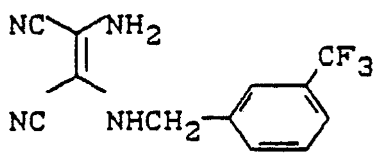

das nach Umkristallisieren aus wenig Chloroform bei 79° C schmilzt.

Beispiel 5

Zu einer Lösung von 17,8 g N-2,6-Dimethyl-propyl-aminomaleinsäurenitril in 100 ml Ethanol abs. gibt man 17,5 g 2,4-Dichlorbenzaldehyd und erhitzt zum Rückfluß. Das Reaktionsende wird durch Dünnschichtchromatographie bestimmt. Man läßt abkühlen und filtriert den Niederschlag ab. Man erhält 31,9 g der folgenden Verbindung mit dem Schmelzpunkt 148° C.

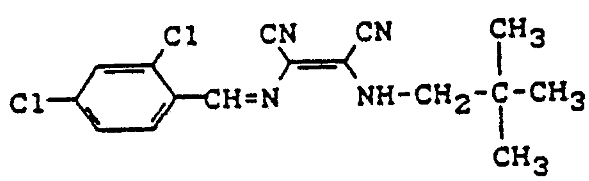

Beispiel 5A

In 100 ml Methanol bringt man eine Mischung aus 10,8 g 2,3-Diaminomaleinsäurenitril und 8,6 g Pivalaldehyd zum Sieden. Die Dünnschichtchromatographie zeigt nach 4 Stunden die Bildung des Azomethins an. Anschließend kühlt man den Reaktionsansatz auf 0 °C ab und gibt portionsweise 4,0 g Natriumborhydrid hinzu. Man läßt 30 Minuten nachrühren. Nach DC-Kontrolle trägt man den Ansatz auf 500 g Eis aus und saugt das ausgefallene Produkt ab. Man erhält 16,3 g der folgenden Verbindung mit dem Schmelzpunkt 129 °C.

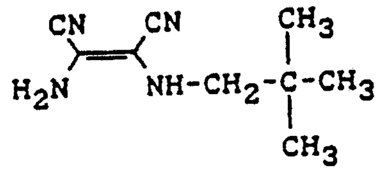

Beispiel 6

Verwendet man im Beispiel 5 2,6-Dichlorbenzaldehyd anstelle von 2,4-Dichlorbenzaldehyd und wendet sonst die gleichen Reaktionsbedingungen an, so erhält man folgende Verbindung mit dem Schmelzpunkt 129 °C.

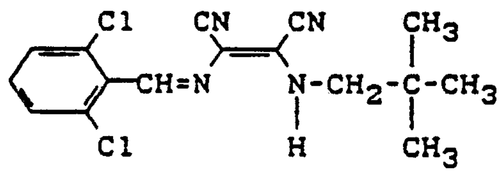

Beispiel 7

In 75 ml Dimethylformamid (DMF) löst man eine Mischung aus 10,8 g 2,3-Diaminomaleinsäurenitril und 18,9 g Pivalaldehyd. Nach der Zugabe von 0,5 g Phosphorpentoxid erwärmt man auf 120 °C (Badtemperatur). Der Umsatz wird durch Dünnschichtchromatographie(DC)-Kontrolle verfolgt. Der Reaktionsansatz wird durch Austragen auf Eis und absaugen des Produkts aufgearbeitet. Man erhält 21,9 g eines braungelben Pulvers der folgenden Formel. Im IR-Spektrum ist oberhalb von 3.100 $cm^{-1}$ keine Schwingung.

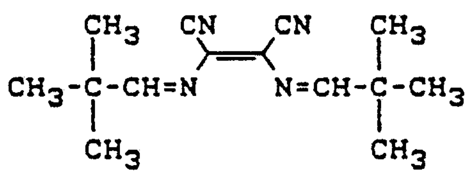

Beispiel 8

33,3 g des nach Beispiel 1 erhaltenen N-2,6-Dichlorbenzyliden-N-pivaloyliden-amino-maleinsäurenitrils werden in 500 ml Methanol in der Hitze gelöst, auf 0 °C abgekühlt und mit 8,0 g Natriumborhydrid reduziert. Die Dünnschichtchromatographie zeigt nach 90 Minuten das Reaktionsende an. Die Aufarbeitung erfolgt durch Austragen auf Eis. Nach der Neutralisation wird das Reaktionsprodukt der folgenden Formel abgesaugt. Man erhält ein amorphes Pulver. Im IR-Spektrum erscheint eine starke Schwingung bei 3.300 $cm^{-1}$.

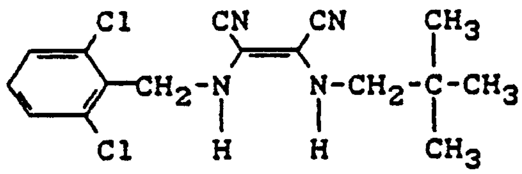

Beispiel 9

Erhitzt man 17,8 g des in Beispiel 5A beschriebenen N-Pivaloyl-amino-maleinsäurenitrils in 100 ml Acetonitril mit 9,8 g Trifluoracetaldehyd so erhält man 23,4 g der folgenden Verbindung als gelbliches Öl ($R_f$-Wert: 0,33/Laufmittel: techn. $CHCl_3$).

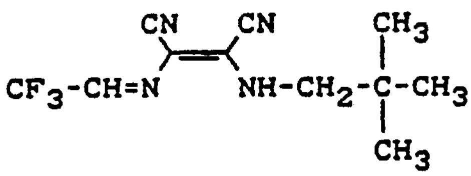

Beispiel 10

Reduziert man 25,8 g der unter Beispiel 9 beschriebenen Verbindung mit 4,0 g Natriumborhydrid in Methanol, so erhält man 23,7 g eines farblosen Öls ($R_f$-Wert: 0,34/Laufmittel: techn. $CHCl_3$) der folgenden Struktur:

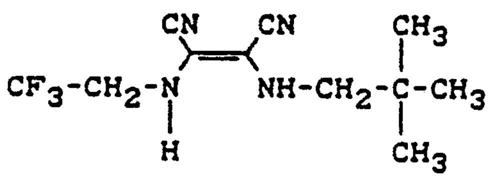

Beispiel 11

In 200 ml Toluol bringt man 13,35 g N-2,6-Dichlorbenzyl-amino-maleinsäurenitril und 8,9 g 2-Hydroxyethyl-trifluormethylsulfon mit 1 g Phosphorpentoxid zum Sieden. Das Reaktionsende wird durch DC-Kontrolle bestimmt. Nach dem Austragen auf Eiswasser und Extraktion mit Chloroform erhält man ein zähes Öl der folgenden Struktur:

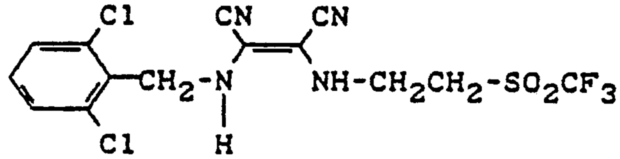

Formulierungsbeispiele

1. Stäubemittel

Zur Herstellung einer zweckmäßigen Wirkstoffzubereitung werden 5 Gew.-Teile Wirkstoff gemäß Beispiel 6 mit 95 Gew.-Teilen eines natürlichen Gesteinsmehl vermengt und staubfein vermahlen. Das so erhaltene Mittel wird in der jeweils gewünschten Menge durch Verstäuben auf die Pflanzen oder ihren Lebensraum aufgebracht.

2. Spritzpulver (dispergierbares Pulver)

Zur Herstellung einer zweckmäßigen Wirkstoffzubereitung werden 50 Gew.-Teile Wirkstoff gemäß Beispiel 1 mit 1 Gew.-Teil Dibutylnaphthalinsulfonat, 4 Gew.-Teilen Ligninsulfonat, 8 Gew.-Teilen hochdisperser Kieselsäure sowie 37 Gew.-Teilen eines natürlichen Gesteinsmehles vermischt und zu einem Pulver vermahlen. Vor der Anwendung wird das benetzbare Pulver mit soviel Wasser verrührt, daß die dabei entstehende Mischung den Wirkstoff in der jeweils gewünschten Konzentration enthält.

3. Emulgierbares Konzentrat

Zur Herstellung einer zweckmäßigen Wirkstoffzubereitung werden 25 Gew.-Teile Wirkstoff gemäß Beispiel 3 in einer Mischung aus 55 Gew.-Teilen Xylol und 10 Gew.-Teilen Cyclohexanon gelöst. Als Emulgator setzt man anschließend 10 Gew.-Teile einer Mischung aus dodecylbenzolsulfonsaurem Calcium und Nonylphenolpolyglykolether hinzu.

Vor der Anwendung wird das Emulsionskonzentrat mit soviel Wasser verdünnt, daß die dabei entstehende Mischung den Wirkstoff in der jeweils gewünschten Konzentration enthält.

4. Granulat

Zur Herstellung einer zweckmäßigen Wirkstoffzubereitung gibt man zu 91 Gew.-Teilen Sand der Körnung 0,5 bis 1,0 mm 2 Gew.-Teile eines Spindelöls und anschließend 7 Gew.-Teile einer feingemahlenen Wirkstoffvermischung, die ihrerseits 75 Gew.-Teile Wirkstoff gemäß Beispiel 6 und 25 Gew.-Teile natürliches Gesteinsmehl enthält. Die Mischung wird so lange in einem geeigneten Mischer behandelt, bis ein gleichmäßiges, frei fließendes und nicht staubendes Granulat entstanden ist. Das Granulat wird in der jeweils gewünschten Menge auf die Pflanzen oder deren Lebensraum verstreut.

**Ansprüche**

1. 2,3-Diaminomaleinsäurenitril-Derivate der allgemeinen Formel

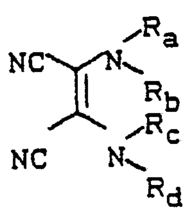

(I)

in welcher

die Substituenten $R_a$, $R_b$, $R_c$ und $R_d$ jeweils eine der folgenden Bedeutungskombinationen A, B, C, D, E oder F annehmen können:

A) $R_a$ und $R_b$ stehen zusammen für den Rest

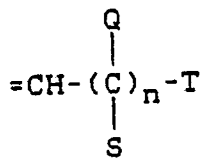

und $R_c$ und $R_d$ stehen zusammen für den Rest

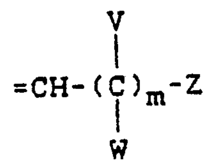

wobei
Q, S, T, V, W und Z gleich oder verschieden sein können und unabhängig voneinander für Wasserstoff, Halcgen, gegebenenfalls substituiertes Alkyl, gegebenenfalls substituiertes Aryl, gegebenenfalls substituiertes Alkylsulfonyl oder gegebenenfalls substituiertes Arylsulfonyl stehen,
n und m unabhängig voneinander die Zahlen 0 bis 10 annehmen können,

B) $R_a$ und $R_b$ stehen zusammen für den Rest

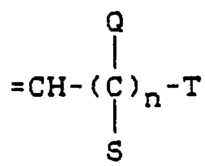

und $R_c$ und $R_d$ stehen zusammen für den Rest

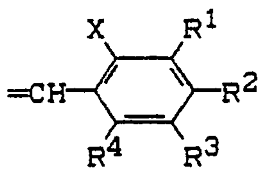

wobei
Q, S, T und n die unter A) angegebene Bedeutung besitzen und
X für Wasserstoff, Halogen, Halogenalkyl oder CN steht,
$R^1$, $R^2$, $R^3$ und $R^4$ gleich oder verschieden sein können und für Wasserstoff, gegebenenfalls substituiertes Alkyl, gegebenenfalls substituiertes Alkoxy, Halogen, CN, $NO_2$, gegebenenfalls substituiertes Dialkylamino, Alkoxycarbonyl, gegebenenfalls substituiertes Alkylthio, gegebenenfalls substituiertes Alkylthionyl, gegebenenfalls substituiertes Alkylsulfonyl, OH, SH, $NH_2$ stehen,

C) $R_a$ steht für Wasserstoff und $R_b$ steht für den Rest

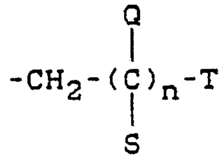

und $R_c$ und $R_d$ stehen zusammen für den Rest

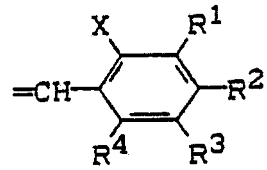

wobei
Q, S, T, n, X, $R^1$, $R^2$, $R^3$ und $R^4$ die unter B) angegebene Bedeutung besitzen,
D) $R_a$ und $R_b$ stehen zusammen für den Rest

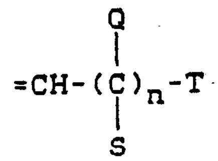

$R_c$ steht für Wasserstoff und $R_d$ steht für den Rest

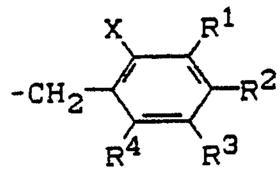

worin
Q, S, T, n, X, $R^1$, $R^2$, $R^3$ und $R^4$ die unter B) angegebene Bedeutung besitzen,
E) $R_a$ und $R_c$ stehen für Wasserstoff, $R_b$ steht für den Rest

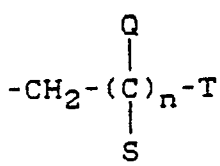

und $R_d$ steht für den Rest

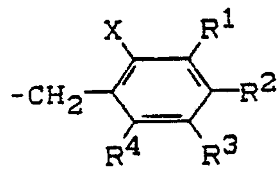

worin
Q, S, T, n, X, $R^1$, $R^2$, $R^3$ und $R^4$ die unter B) angegebene Bedeutung besitzen,
F) $R_a$ und $R_c$ stehen für Wasserstoff, $R_b$ steht für den Rest

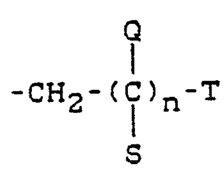

und $R_d$ für den Rest

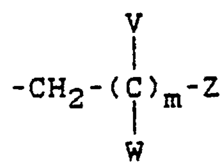

wobei
Q, S, T, V, W, Z, n und m die unter A) angegebene Bedeutung besitzen.

2. 2,3-Diaminomaleinsäurenitril-Derivate der Formeln ($I_A$) bis ($I_F$):

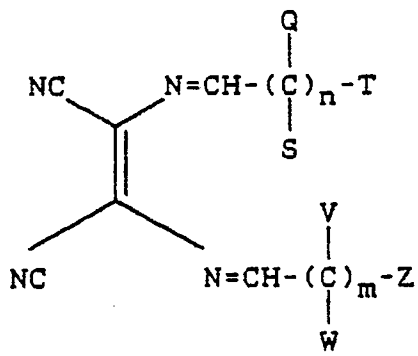

($I_A$)

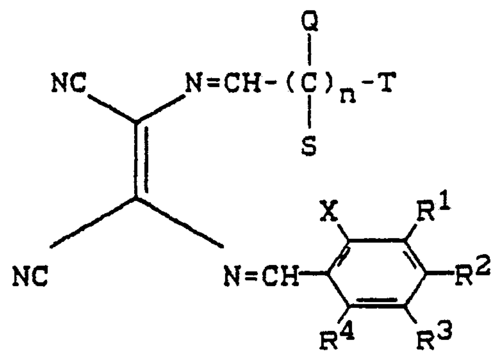

($I_B$)

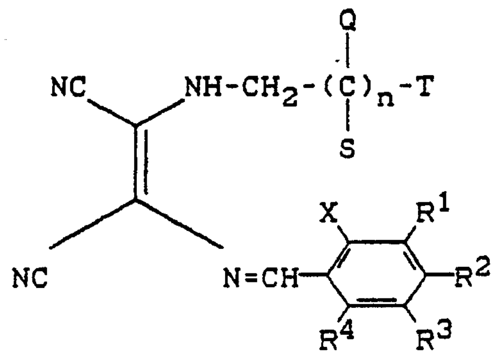

($I_C$)

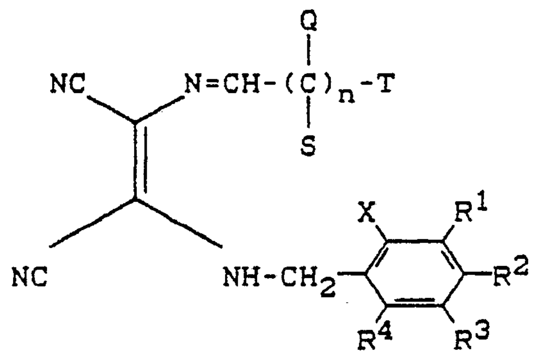

($I_D$)

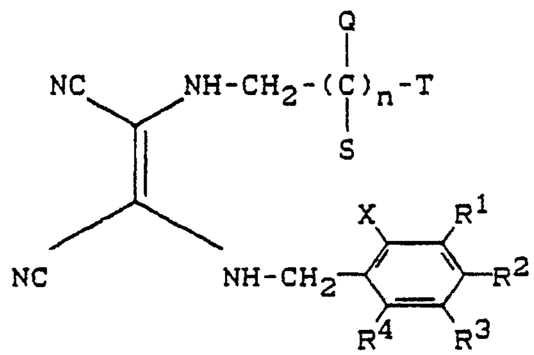

($I_E$)

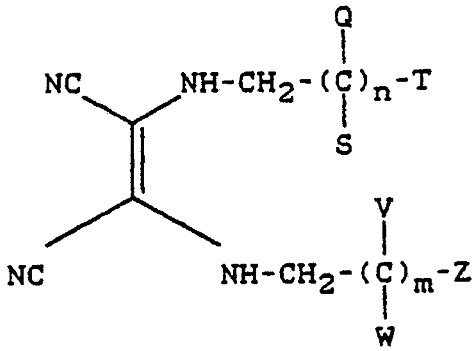

($I_F$)

wobei in den Formeln ($I_A$) bis ($I_F$)
Q, S, T, V, W und Z gleich oder verschieden sein können und unabhängig voneinander für Wasserstoff, Halogen, gegebenenfalls substituiertes Alkyl, gegebenenfalls substituiertes Aryl, gegebenenfalls substituiertes Alkylsulfonyl oder gegebenenfalls substituiertes Arylsulfonyl stehen,
n und m unabhängig voneinander die Zahlen 0 bis 10 annehmen können,
X für Wasserstoff, Halogen, Halogenalkyl oder CN steht,
$R^1$, $R^2$, $R^3$ und $R^4$ gleich oder verschieden sein können und
für Wasserstoff, gegebenenfalls substituiertes Alkyl, gegebenenfalls substituiertes Alkoxy, Halogen, CN, $NO_2$, gegebenenfalls substituiertes Dialkylamino, Alkoxycarbonyl, gegebenenfalls substituiertes Alkylthio, gegebenenfalls substituiertes Alkylthionyl, gegebenenfalls substituiertes Alkylsulfonyl, OH, SH, $NH_2$ stehen.

3. Verfahren zur Herstellung von 2,3-Diaminomaleinsäurenitril-Derivaten der Formel ($I_A$):

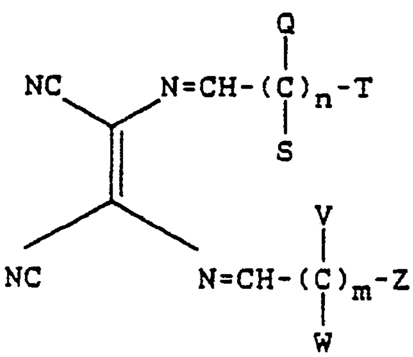

($I_A$)

wobei
Q, S, T, V, W und Z gleich oder verschieden sein können und unabhängig voneinander für Wasserstoff, Halogen, gegebenenfalls substituiertes Alkyl, gegebenenfalls substituiertes Aryl, gegebenenfalls substituiertes Alkylsulfonyl oder gegebenenfalls substituiertes Arylsulfonyl stehen,
n und m unabhängig voneinander die Zahlen 0 bis 10 annehmen können,
dadurch gekennzeichnet, daß man 2,3-Diaminomaleinsäurenitril der Formel (II)

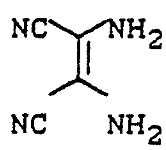

(II)

in äquimolaren Mengen in einem Verdünnungsmittel mit einem Aldehyd der Formel (III)

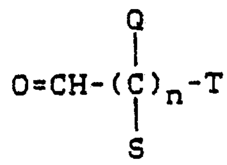

(III)

in welcher Q, S, T und n die oben angegebene Bedeutung haben, zum Azomethin der Formel (IV)

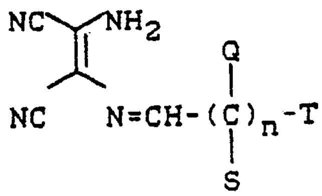

(IV)

umsetzt und dieses dann unter Zusatz eines Katalysators mit molarer Menge eines weiteren Aldehyds der Formel (V)

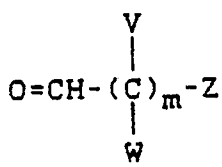

(V)

in welcher V, W, Z und m die oben angegebene Bedeutung besitzen, umsetzt, oder daß man zum Erhalt der symmetrischen Bisazomethine der Formel (VI)

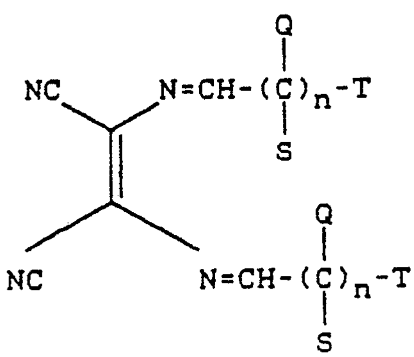

(VI)

1 Mol 2,3-Diaminomaleinsäurenitril der Formel (II)

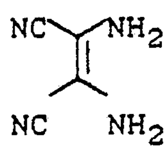

(II)

in einem Verdünnungsmittel unter Zusatz eines Katalysators mit 2 Mol eines Aldehyds der Formel (III)

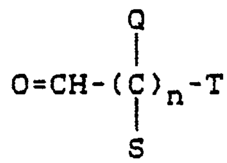 (III)

in welcher Q, S, T und n die obengenannte Bedeutung haben, umsetzt.

4. Verfahren zur Herstellung von 2,3-Diaminomaleinsäurenitril-Derivaten der Formel ($I_B$)

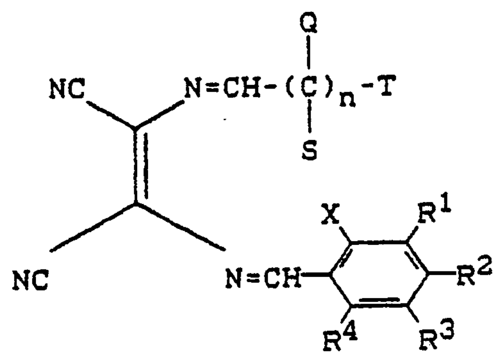 ($I_B$)

in welcher

Q, S, T gleich oder verschieden sein können und unabhängig voneinander für Wasserstoff, Halogen, gegebenenfalls substituiertes Alkyl, gegebenenfalls substituiertes Aryl, gegebenenfalls substituiertes Alkylsulfonyl oder gegebenenfalls substituiertes Arylsulfonyl stehen,

n für die Zahl 0 bis 10 steht,

X für Wasserstoff, Halogen, Halogenalkyl oder CN steht,

$R^1$, $R^2$, $R^3$ und $R^4$ gleich oder verschieden sein können und für Wasserstoff, gegebenenfalls substituiertes Alkyl, gegebenenfalls substituiertes Alkoxy, Halogen, CN, $NO_2$, gegebenenfalls substituiertes Dialkylamino, Alkoxycarbonyl, gegebenenfalls substituiertes Alkylthio, gegebenenfalls substituiertes Alkylthionyl, gegebenenfalls substituiertes Alkylsulfonyl, OH, SH, $NH_2$, stehen,

dadurch gekennzeichnet, daß man 2,3-Diaminomaleinsäurenitril der Formel (II)

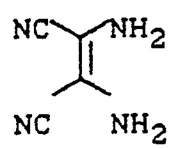 (II)

in äquimolaren Mengen in einem Verdünnungsmittel mit einem Aldehyd der Formel (III)

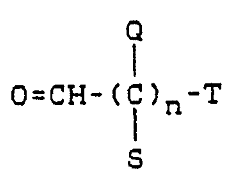 (III)

oder einem Aldehyd der Formel (VII)

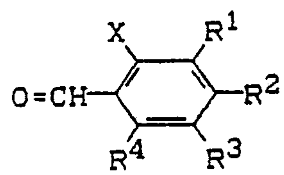 (VII)

wobei in den Formeln (III) und (VII) Q, S, T, n, X, $R^1$, $R^2$, $R^3$ und $R^4$ die oben angegebene Bedeutung besitzen, beim Umsatz mit (III) zum Azomethin der Formel (IV)

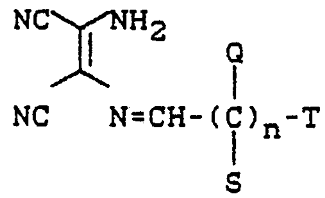

(IV)

bzw. beim Umsatz mit (VII) zum Azomethin der Formel (VIII)

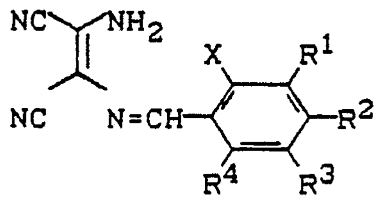

(VIII)

umsetzt,
und diese Reaktionsprodukte im Fall von (IV)
unter Zusatz eines Katalysators mit molarer Menge eines Aldehyds der Formel (VII)

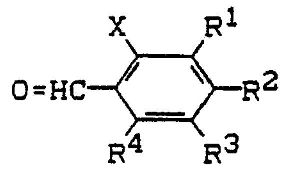

(VII)

bzw. im Fall von (VIII)
unter Zusatz eines Katalysators mit molarer Menge eines Aldehyds der Formel (III)

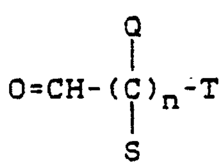

(III)

in welcher Q, S, T, n, X, $R^1$, $R^2$, $R^3$ und $R^4$ die oben angegebene Bedeutung haben, umsetzt.

5. Verfahren zur Herstellung von 2,3-Diaminomaleinsäurenitril-Derivaten der Formel ($I_C$)

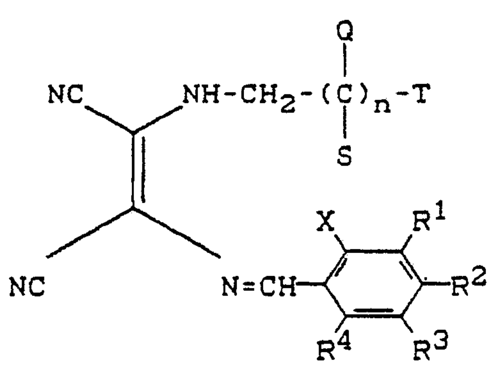

($I_C$)

in welcher
Q, S, T gleich oder verschieden sein können und unabhängig voneinander für Wasserstoff, Halogen, gegebenenfalls substituiertes Alkyl, gegebenenfalls substituiertes Aryl, gegebenenfalls substituiertes Alkylsulfonyl oder gegebenenfalls substituiertes Arylsulfonyl stehen,
n für die Zahl 0 bis 10 steht,
X für Wasserstoff, Halogen, Halogenalkyl oder CN steht,
$R^1$, $R^2$, $R^3$ und $R^4$ gleich oder verschieden sein können und
für Wasserstoff, gegebenenfalls substituiertes Alkyl, gegebenenfalls substituiertes Alkoxy, Halogen, CN, $NO_2$, gegebenenfalls substituiertes Dialkylamino, Alkoxycarbonyl, gegebenenfalls substituiertes Alkylthio, gegebenenfalls substituiertes Alkylthionyl, gegebenenfalls substituiertes Alkylsulfonyl, OH, SH, $NH_2$ stehen,
dadurch gekennzeichnet, daß man 2,3-Diaminomaleinsäurenitril-Derivate der allgemeinen Formel (IX)

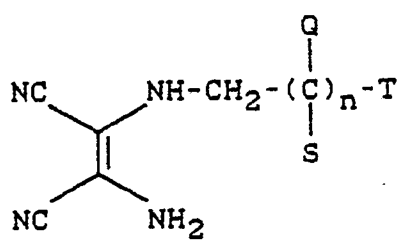

(IX)

in welcher
Q, S, T und n die oben angegebene Bedeutung besitzen,
in äquimolarer Menge in einem geeigneten Verdünnungsmittel mit einem Aldehyd der Formel (VII)

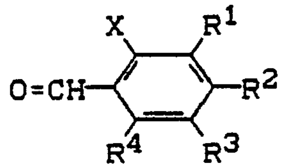

(VII)

in welcher X, $R^1$, $R^2$, $R^3$ und $R^4$ die oben angegebene Bedeutung besitzen, umsetzt.

6. Verfahren zur Herstellung von 2,3-Diaminomaleinsäurenitril-Derivate der Formel ($I_D$)

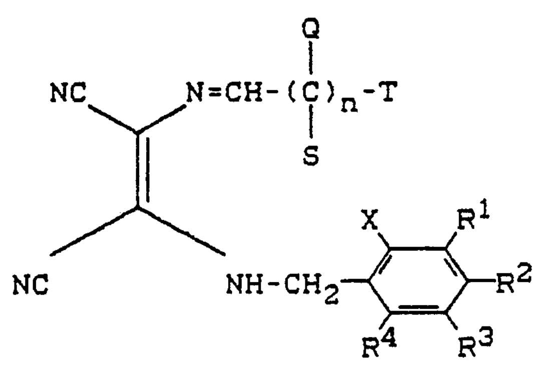

($I_D$)

in welcher
Q, S, T gleich oder verschieden sein können und unabhängig voneinander für Wasserstoff, Halogen, gegebenenfalls substituiertes Alkyl, gegebenenfalls substituiertes Aryl, gegebenenfalls substituiertes Alkylsulfonyl oder gegebenenfalls substituiertes Arylsulfonyl stehen,
n für die Zahl 0 bis 10 steht,
X für Wasserstoff, Halogen, Halogenalkyl oder CN steht,
$R^1$, $R^2$, $R^3$ und $R^4$ gleich oder verschieden sein können und
für Wasserstoff, gegebenenfalls substituiertes Alkyl, gegebenenfalls substituiertes Alkoxy, Halogen, CN, $NO_2$, gegebenenfalls substituiertes Dialkylamino, Alkoxycarbonyl, gegebenenfalls substituiertes Alkylthio, gegebenenfalls substituiertes Alkylthionyl, gegebenenfalls substituiertes Alkylsulfonyl, OH, SH, $NH_2$ stehen,

dadurch gekennzeichnet, daß man 2,3-Diaminomaleinsäurenitril-Derivate der allgemeinen Formel (X)

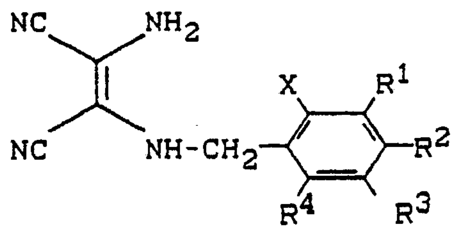

(X)

in welcher
X, $R^1$, $R^2$, $R^3$ und $R^4$ die oben angegebene Bedeutung haben, in äquimolarer Menge in einem geeigneten Verdünnungsmittel mit einem Aldehyd der Formel (III)

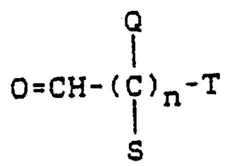

(III)

in welcher Q, S, T und n die oben angegebene Bedeutung besitzen, umsetzt.

7. Verfahren zur Herstellung von 2,3-Diaminomaleinsäurenitril-Derivaten der Formel ($I_E$)

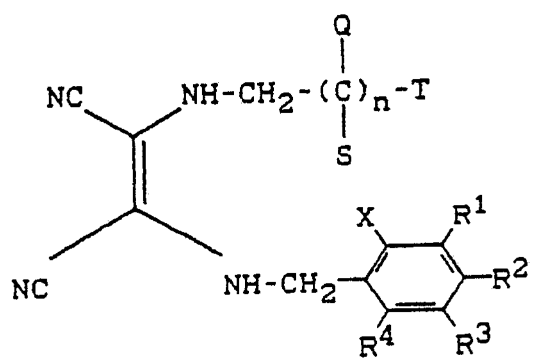

($I_E$)

in welcher
Q, S, T gleich oder verschieden sein können und unabhängig voneinander für Wasserstoff, Halogen, gegebenenfalls substituiertes Alkyl, gegebenenfalls substituiertes Aryl, gegebenenfalls substituiertes Alkylsulfonyl oder gegebenenfalls substituiertes Arylsulfonyl stehen,
n für die Zahl 0 bis 10 steht,
X für Wasserstoff, Halogen, Halogenalkyl oder CN steht,
$R^1$, $R^2$, $R^3$ und $R^4$ gleich oder verschieden sein können und
für Wasserstoff, gegebenenfalls substituiertes Alkyl, gegebenenfalls substituiertes Alkoxy, Halogen, CN, $NO_2$, gegebenenfalls substituiertes Dialkylamino, Alkoxycarbonyl, gegebenenfalls substituiertes Alkylthio, gegebenenfalls substituiertes Alkylthionyl, gegebenenfalls substituiertes Alkylsulfonyl, OH, SH, $NH_2$ stehen,
dadurch gekennzeichnet, daß man ein 2,3-Diaminomaleinsäurenitril-Derivat der Formeln ($I_B$), ($I_C$) oder ($I_D$)

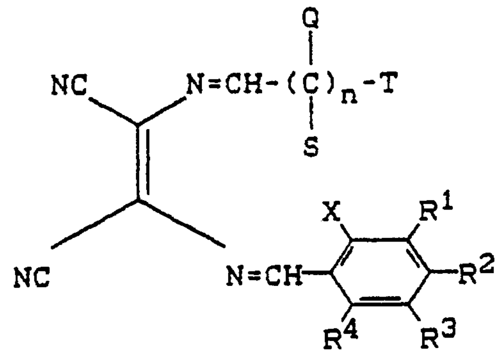

$(I_B)$

und/oder

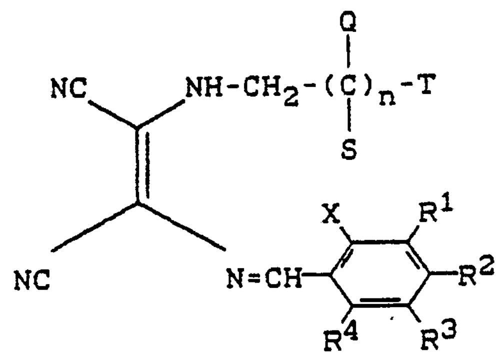

$(I_C)$

und/oder

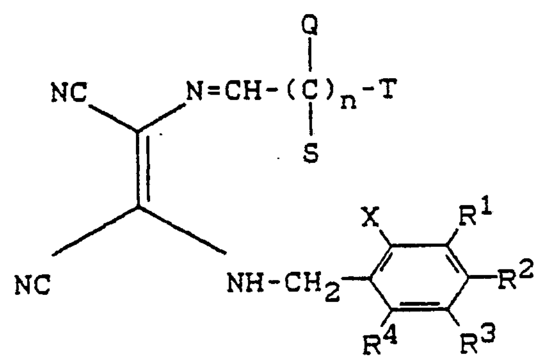

$(I_D)$

in welchen Q, S, T, n, X, $R^1$, $R^2$, $R^3$ und $R^4$ die oben angegebene Bedeutung besitzen, in einem geeigneten Lösungsmittel reduziert.

8. Verfahren zur Herstellung von 2,3-Diaminomaleinsäurenitril-Derivaten der Formel $(I_F)$

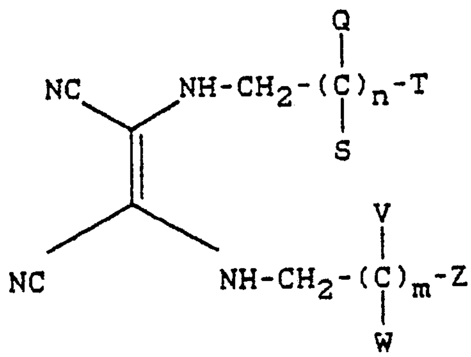

(I$_F$)

in welcher
Q, S, T, V, W und Z gleich oder verschieden sein können und unabhängig voneinander für Wasserstoff, Halogen, gegebenenfalls substituiertes Alkyl, gegebenenfalls substituiertes Aryl, gegebenenfalls substituiertes Alkylsulfonyl oder gegebenenfalls substituiertes Arylsulfonyl stehen,
n und m unabhängig voneinander die Zahlen 0 bis 10 annehmen können,
dadurch gekennzeichnet, daß man ein 2,3-Diaminomaleinsäurenitril-Derivat der Formeln (I$_A$), (XIII) oder (XIV)

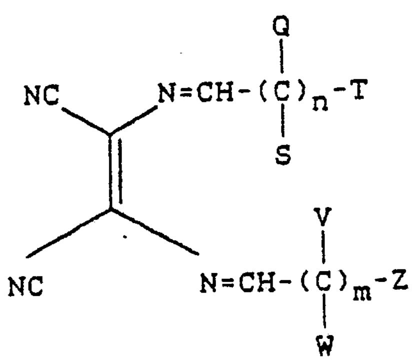

(I$_A$)

und/oder

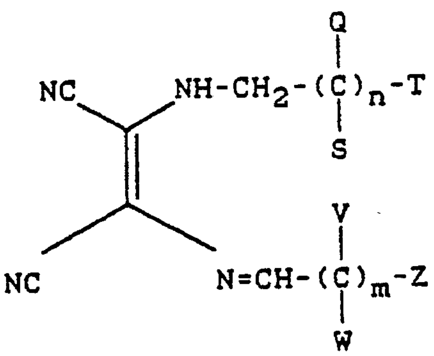

(XIII)

und/oder

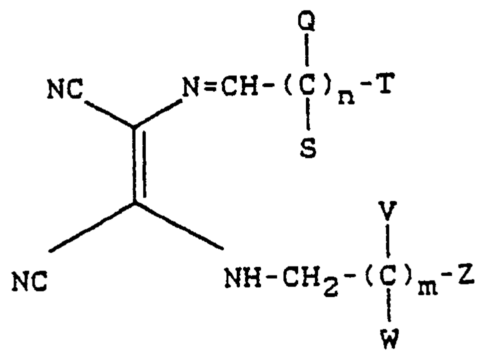

(XIV)

in welchen Q, S, T, V, W, Z, n und m die oben angegebene Bedeutung besitzen, in einem geeigneten Verdünnungsmittel reduziert.

9. Schädlingsbekämpfungsmittel, gekennzeichnet durch einen Gehalt an mindestens einem 2,3-Diaminomaleinsäurenitril-Derivat der Formel (I).

10. Akarizides Mittel, gekennzeichnet durch einen Gehalt an mindestens einem 2,3-Diaminomaleinsäurenitril-Derivat der Formel (I).

11. Verfahren zur Bekämpfung von Spinnentieren, dadurch gekennzeichnet, daß man Derivate des 2,3-Diaminomaleinsäurenitrils der allgemeinen Formel (I) gemäß Anspruch 1 auf Spinnentiere und/oder ihren Lebensraum einwirken läßt.

12. Verwendung von Derivaten des 2,3-Diaminomaleinsäurenitrils der allgemeinen Formel (I) gemäß Anspruch 1 zur Bekämpfung von Spinnentieren.

13. Verfahren zur Herstellung von akariziden Mitteln, dadurch gekennzeichnet, daß man Derivate des 2,3-Diaminomaleinsäurenitrils der allgemeinen Formel (I) gemäß Anspruch 1 mit Streckmitteln und/oder oberflächenaktiven Mitteln vermischt.

14. Verbindungen der allgemeinen Formel (IXA)

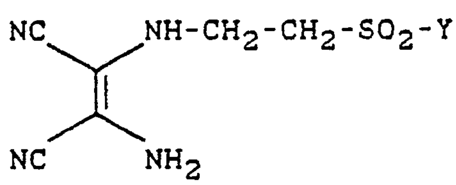

(IXA)

in welcher

Y für gegebenenfalls substituiertes Alkyl oder Aryl steht.

15. Verfahren zur Herstellung von Verbindungen der allgemeinen Formel (IXA)

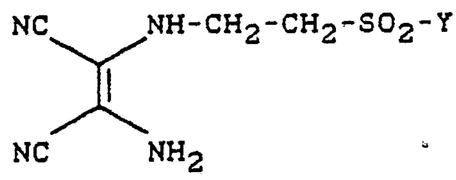

(IXA)

in welcher
Y für gegebenenfalls substituiertes Alkyl oder Aryl steht, dadurch gekennzeichnet, daß man 2,3-Diaminomaleinsäurenitril der Formel (II)

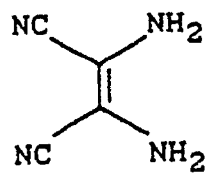

(II)

in einem geeigneten Lösungsmittel mit einem Vinylsulfon bzw. einer Vinylsulfon abspaltenden Verbindung

$CH_2 = CH\text{-}SO_2\text{-}Y$ (XI) oder

$L\text{-}CH_2\text{-}CH_2\text{-}SO_2\text{-}Y$ (XII)

wobei L = Abgangsgruppe ist, umsetzt.

Europäisches Patentamt

# EUROPÄISCHER RECHERCHENBERICHT

Nummer der Anmeldung

EP 88 11 6980

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,X | US-A-4 002 616 (J.F. NEUMER)<br>* Beispiele; Spalte 2 * | 1-6 | C 07 C 121/84<br>C 07 C 121/45<br>C 07 C 147/02<br>A 01 N 37/34<br>A 01 N 41/10 |
| X | JOURNAL OF THE AMERICAN CHEMICAL SOCIETY, Band 98, Nr. 5, 3. März 1976, Seiten 1099-1103; J.H. BOYER et al.: "N-Alkylformimidoyl cyanides and isocyanides"<br>* Tabelle V * | 1,2 | |
| D,X | THE JOURNAL OF ORGANIC CHEMISTRY, Band 39, Nr. 16, 9. August 1974, Seiten 2341-2350; R.W. BEGLAND et al.: "Hydrogen cyanide chemistry. VIII. New chemistry of diaminomaleonitrile. Heterocyclic synthesis"<br>* Seite 2341 * | 1,2,5-8 | |
| A | PATENT ABSTRACTS OF JAPAN, Band 1, Nr. 29, 28. März 1977, Seite 1678 C 76; & JP-A-51 151 325 (KUMIAI CHEMICAL INDUSTRY K.K.) 25-12-1976 | 9-13 | **RECHERCHIERTE SACHGEBIETE (Int. Cl.4)**<br>C 07 C 121/00<br>C 07 C 147/00<br>A 01 N 37/00 |

Der vorliegende Recherchenbericht wurde für alle Patentansprüche erstellt

| Recherchenort | Abschlußdatum der Recherche | Prüfer |
|---|---|---|
| DEN HAAG | 09-02-1989 | WRIGHT M.W. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)